# EUROPEAN PATENT APPLICATION

(11) **EP 4 379 044 A1**
(43) Date of publication of application: **05.06.2024**
(21) Application number: 22849022.3
(22) Date of filing: 31.05.2022
(51) Int. Cl.: C12N 5/079, C07K 14/78, C12M 1/00, C12N 1/00

(54) **CELL CULTURE METHOD, CELL CULTURE CONTAINER, METHOD FOR PRODUCING CELL CULTURE CONTAINER, AND CELL-CONTAINING STRUCTURE**

(30) Priority: 30.07.2021 JP 2021125556; 18.03.2022 JP 2022044329
(71) Applicant: Ricoh Company, Ltd., Tokyo 143-8555 (JP)
(72) Inventor: SHIONOIRI, Momoko, Tokyo 143-8555 (JP); ARATANI, Tomoyuki, Tokyo 143-8555 (JP); IJICHI, Rie, Tokyo 143-8555 (JP); SAMESHIMA, Tatsuya, Tokyo 143-8555 (JP); KOSHIZUKA, Shinnosuke, Tokyo 143-8555 (JP); KITAZAWA, Tomofumi, Tokyo 143-8555 (JP); NAKAYAMA, Tomoaki, Tokyo 143-8555 (JP); KAGA, Yuki, Tokyo 143-8555 (JP)
(74) Representative: Marks & Clerk LLP
(86) International application number: PCT/JP2022/022134
(87) International publication number: WO 2023/007943

(57) **Abstract**

A cell culture method includes a step of performing adherent culture of cells on a coat layer layered on a surface of a cell culture container by placing a culture medium and the cells in the cell culture container, in which the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with the cells, or the coat layer includes the layer (i) and the layer (i) is disposed at a position at which the layer (i) comes into contact with the cells, and the culture medium includes the cell adhesion factor.

## Description

### [Technical Field]

The present invention relates to a cell culture method, a cell culture container, a method for producing a cell culture container, and a cell-containing structure.

Priority is claimed on Japanese Patent Application No. 2021-125556, filed July 30, 2021 and Japanese Patent Application No. 2022-044329, filed March 18, 2022, the content of which is incorporated herein by reference.

### [Background Art]

In recent years, research on neural regenerative medicine has been actively conducted with a goal of developing regenerative medicine for the nervous system. In this research, it is necessary to investigate the functional evaluation or pharmacological effects of cell aggregates including nerve cells. Among the above, the functional evaluation of neural networks formed of aggregates of nerve cells is particularly important for obtaining electrophysiological knowledge.

Currently, functional evaluation of neural networks is performed not only by detecting Ca concentration changes and observing synaptic activities in general-purpose flat culture plates, but also by extracellular potential measurement using planar microelectrode arrays (MEA). In order to perform functional evaluation of these neural networks, it is necessary to culture nerve cell aggregates for a long period of time. Furthermore, in the case of human iPS cell-derived nerve cells, it is necessary to culture the nerve cells at a higher density and for longer periods than for nerve cells derived from non-human animals.

On the other hand, it is known that nerve cell aggregates tend to peel from the culture surface of the culture container during long-term culturing. This peeling is a major factor causing variations in the results of functional evaluation of nerve cell aggregates and pharmacological effects.

Generally, in order to suppress the peeling of nerve cell aggregates from the culture surface of the culture container, the culture surface of the culture container is coated with a base (polycationic material) before culturing and then a cell adhesion factor is coated on the base. These procedures have been disclosed by companies that market nerve cells and companies that market MEA measurement devices.

Furthermore, in a case where the effect of coating the base and cell adhesion factor alone is not sufficient, a method for exposing the culture surface to ozone plasma or a method for performing a pre-process such as immersion in a concentrated sterilized protein solution, before coating the base and cell adhesion factor, have also been disclosed.

For example, Non Patent Document 1 discloses a method for exposing the culture surface of a cell culture container to ozone plasma for 1 minute. However, performing this method requires an expensive ozone plasma device. Furthermore, there may be problems in that the culture surface is extremely hydrophilic, causing the cell suspension to wet and spread over the entire surface when seeding the cells and in that a large quantity of cells is required in the case of MEA and the cells may be seeded even onto a reference electrode.

Non Patent Document 2 discloses a method in which a culture surface is immersed in a concentrated sterilized protein solution. However, as the concentrated sterilized protein solution, only a serum culture medium, an albumin solution, and the like are described and the conditions such as the concentration of the concentrated sterilized protein solution are not described and are not clear.

Non Patent Document 3 discloses a method in which a culture surface is immersed in a serum culture medium for 1 to 2 minutes. This method certainly has an effect of suppressing the peeling of cells from the culture surface. However, as a result of verification by the present inventors, it was found that there is room for further improvement in order to suppress the peeling of cell aggregates including nerve cells and reduce variations in the results of functional evaluation.

In addition, methods were also disclosed in which the base material itself of a cell culture container is made to have characteristics that suppress cell peeling. For example, Patent Document 1 describes a cell culture container having a layered membrane in which a collagen layer is provided on a silicone rubber membrane. However, this method is impractical due to cost and is not applicable to existing cell culture containers that are generally widely distributed.

### [Summary of Invention]

### [Technical Problem]

An object of the present invention is to provide a cell culture method, a cell culture container, and a method for producing the cell culture container, with which it is possible to suppress the peeling of cell aggregates from a culture surface and to obtain stable functional evaluation results.

### [Solution to Problem]

A cell culture method according to the present invention includes a step of performing adherent culture of cells on a coat layer layered on a surface of a cell culture container by placing a culture medium and the cells in the cell culture container, in which the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with the cells, or the coat layer includes the layer (i), the layer (i) is disposed at a position at which the layer (i) comes into contact with the cells, and the culture medium includes the cell adhesion factor.

In a cell culture container according to the present invention, a coat layer is layered on a surface, the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with cells during cell culture.

A method for producing a cell culture container having a modified culture surface according to the present invention includes a step 1 of bringing a solution including gelatin or casein into contact with a surface of the cell culture container, a step 2 of removing the solution including the gelatin or the casein, a step 3 of bringing a solution including a polycationic material into contact with the surface of the cell culture container, a step 4 of removing the solution including the polycationic material, a step 5 of bringing a solution including a cell adhesion factor into contact with the surface of the cell culture container, and a step 6 of removing the solution including the cell adhesion factor, in which the production method is performed in the above order, or in an order of the step 3, the step 4, the step 1, the step 2, the step 5, and the step 6.

A cell-containing structure according to the present invention includes a cell culture container, cells, and a culture medium, in which the culture medium and the cells are placed in the cell culture container, the cell culture container has a coat layer layered on a surface and the cells are adhered on the coat layer, the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with the cells, or the coat layer includes the layer (i), the layer (i) is disposed at a position at which the layer (i) comes into contact with the cells, and the culture medium includes the cell adhesion factor.

### [Advantageous Effects of Invention]

According to the present invention, it is possible to provide a cell culture method, a cell culture container, and a method for producing the cell culture container, with which it is possible to suppress the peeling of cell aggregates from a culture surface and obtain stable functional evaluation results.

### [Brief Description of Drawings]

FIGS. 1(a) to 1(d) are schematic diagrams of a cell culture method according to one embodiment.
FIGS. 2(a) and 2(b) are schematic cross-sectional diagrams of a cell culture container according to one embodiment.
FIGS. 3(a) to 3(h) are representative photographs capturing cell aggregates cultured in the cell culture containers of Examples 1 and 2 and Comparative Examples 1 and 2.
FIG. 4 is a diagram showing representative images capturing cell aggregates cultured in the cell culture containers of Example 3 and Comparative Examples 3 and 4 and transported by air, as well as displaying the number of effective electrodes that detected action potentials.
FIGS. 5(a) to 5(c) are graphs showing variations (CV values) in the spontaneous firing rate, burst count, and synchronous burst count of cell aggregates cultured in cell culture containers of Example 3 and Comparative Examples 3 and 4 and transported by air.
FIGS. 6(a) to 6(c) are graphs showing variations (CV values) in the spontaneous firing rate, burst count, and synchronous burst count of cell aggregates cultured in cell culture containers of Example 3 and Comparative Examples 3 to 5 and allowed to stand overnight in a room temperature environment.
FIGS. 7(a) and 7(b) are representative images capturing cell aggregates cultured in the cell culture containers of Example 4 and Comparative Example 6.
FIGS. 8(a) and 8(b) are mapping images of effective electrodes that detected action potentials of cell aggregates cultured in cell culture containers of Example 4 and Comparative Example 6, and are images that display the ratios of the number of effective electrodes.
FIG. 9 shows representative images capturing cell aggregates cultured in the cell culture containers of Example 5 and Comparative Example 7.
FIGS. 10(a) to 10(d) are representative photographs capturing cell aggregates cultured in the cell culture containers of Example 6 and Comparative Example 8.
FIG. 11 shows representative photographs capturing cell aggregates cultured in the cell culture containers of Examples 6 and 7 and Comparative Examples 8 to 16.
FIG. 12 shows representative photographs capturing cell aggregates cultured in the cell culture containers of Examples 6 and 7 and Comparative Examples 8 to 13.
FIG. 13 shows representative photographs capturing cell aggregates cultured in the cell culture containers of Example 10 and Comparative Example 21.

### [Description of Embodiments]

A description will be given below of embodiments of the present invention with reference to the drawings depending on the case. The dimensional ratios in each diagram may be partially exaggerated for the sake of explanation and do not necessarily match the actual dimensional ratios.

### [Cell Culture Method Using Modified Culture Surface]

The cell culture method in one embodiment includes a step of performing adherent culture of cells on a coat layer layered on a surface of a cell culture container by placing a culture medium and the cells in the cell culture container, in which the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with the cells, or the coat layer includes the layer (i), the layer (i) is disposed at a position at which the layer (i) comes into contact with the cells, and the culture medium includes the cell adhesion factor.

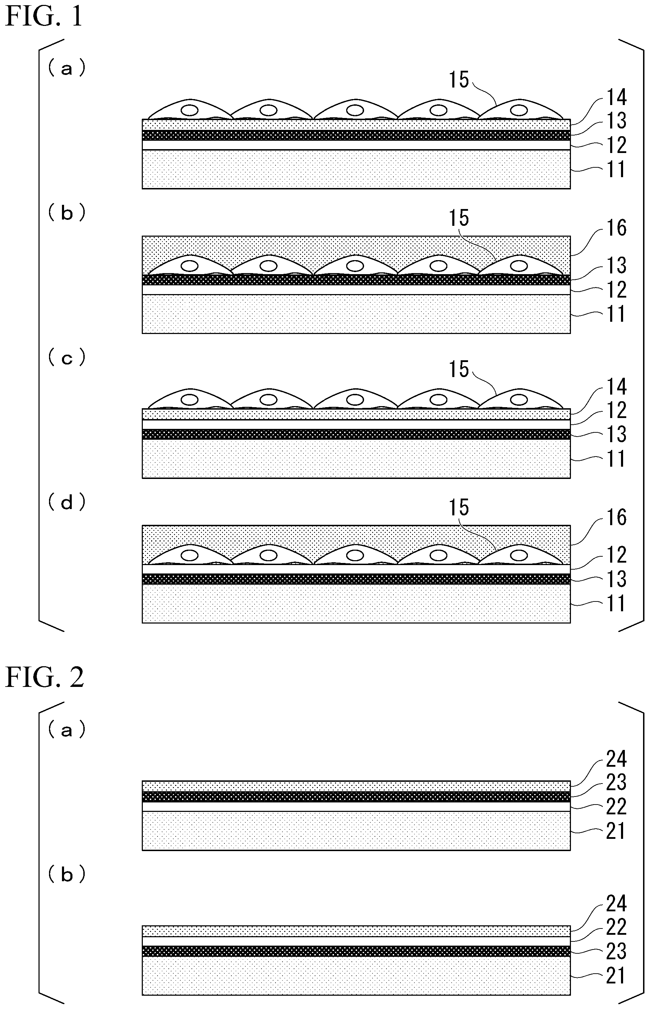

FIGS. 1(a) to 1(d) are schematic diagrams each showing an example of the cell culture method of the present embodiment. The cell culture method shown in FIG. 1(a) is an example of a method in which a culture medium and cells are placed in a cell culture container and adherent culture of the cells is performed on a coat layer layered on the surface of the cell culture container. In this example, the coat layer is layered on the surface of the cell culture container and adherent culture of the cells is performed on the coat layer. On the surface of a base material 11 of the cell culture container, a layer 12 including gelatin or casein, a layer 13 including a polycationic material, and a layer 14 including a cell adhesion factor are layered in this order. Cells 15 are adhered to the layer 14.

The cell culture method shown in FIG. 1(b) is an example of a method in which a culture medium and cells are placed in a cell culture container and adherent culture of the cells is performed on a coat layer layered on the surface of the cell culture container. In this example, the coat layer is layered on the surface of the cell culture container and adherent culture of the cells is performed on the coat layer. On the surface of the base material 11 of the cell culture container, the layer 12 including gelatin or casein and the layer 13 including a polycationic material are layered in this order. The cells 15 are adhered to the layer 13. In addition, a culture medium 16 includes a cell adhesion factor.

The cell culture method shown in FIG. 1(c) is an example of a method in which a culture medium and cells are placed in a cell culture container and adherent culture of the cells is performed on a coat layer layered on the surface of the cell culture container. In this example, the coat layer is layered on the surface of the cell culture container and adherent culture of the cells is performed on the coat layer. On the surface of the base material 11 of the cell culture container, the layer 13 including a polycationic material, the layer 12 including gelatin or casein, and the layer 14 including a cell adhesion factor are layered in this order. The cells 15 are adhered to the layer 14.

The cell culture method shown in FIG. 1(d) is an example of a method in which a culture medium and cells are placed in a cell culture container and adherent culture of the cells is performed on a coat layer layered on the surface of the cell culture container. In this example, the coat layer is layered on the surface of the cell culture container and adherent culture of the cells is performed on the coat layer. On the surface of the base material 11 of the cell culture container, the layer 13 including a polycationic material, and the layer 12 including gelatin or casein are layered in this order. The cells 15 are adhered to the layer 12. In addition, the culture medium 16 includes a cell adhesion factor.

The coat layer is layered on the surface of the base material of the cell culture container. The coat layer includes the layer (i). The layer (i) includes the layer (i-1) including gelatin or casein and the layer (i-2) that includes a polycationic material. The arrangement of these layers is in the order of the base material of the cell culture container, the layer (i-1), and the layer (i-2), or in the order of the base material of the cell culture container, the layer (i-2), and the layer (i-1).

In a case where the layers are disposed in the order of the base material of the cell culture container, the layer (i-1), and the layer (i-2), it is preferable that the base material and layer (i-1) are adjacent to each other and no other substances are present between the surface of the base material and the layer (i-1). In addition, it is preferable that the layer (i-1) and the layer (i-2) are adjacent to each other and no other substances are present between the layer (i-1) and the layer (i-2).

In a case where the layers are disposed in the order of the base material of the cell culture container, the layer (i-2), and the layer (i-1), it is preferable that the base material and layer (i-2) are adjacent to each other and no other substances are present between the surface of the base material and the layer (i-2). In addition, it is preferable that the layer (i-2) and the layer (i-1) are adjacent to each other and no other substances are present between the layer (i-2) and the layer (i-1).

The layer (i) may be disposed to be in contact with the cells and the culture medium may contain a cell adhesion factor. In such a case, it is preferable that the layer (i) and the cells are adjacent to each other and no other substances are present between the layer (i) and the cells.

The layer (ii) including a cell adhesion factor may be further layered on the layer (i). In such a case, the arrangement of these layers is in the order of the base material of the cell culture container, the layer (i), and the layer (ii). It is preferable that the layer (i) and the layer (ii) are adjacent to each other and no other substances are present between the layer (i) and the layer (ii). In such a case, the layer (ii) is disposed in contact with the cells. In addition, it is preferable that the layer (ii) and the cells are adjacent to each other and no other substances are present between the layer (ii) and the cells.

As described below in the Examples, the present inventors found that a cell culture method in which a coat layer having the configuration described above is layered on the surface of a cell culture container and adherent culture of the cells is performed on the coat layer makes it possible to significantly suppress the peeling of the cells from the culture surface even in a case where the cells are easily peelable cells.

Accordingly, the cell culture method of the present embodiment is particularly suitable for culturing easily peelable cells. Examples of easily peelable cells include cells that are peeled from the culture surface of a cell culture container simply by the application of shocks or vibration to the cell culture container. Specific examples of easily peelable cells include cell aggregates including nerve cells, HEK293, which is a human embryonic kidney cell line, and the like and cells that are as easily or more easily peeled than such cells (peeling occurs with the same degree of shock or vibration or less) are encompassed in the "easily peelable cells" of the present disclosure.

When the peeling of cell aggregates including nerve cells from the culture surface is suppressed, it is possible to stably detect and evaluate the action potentials of nerve cells using MEA or the like. In addition, it is also possible to obtain stable evaluation results by other means for analyzing, such as detection of changes in Ca concentration, observation of synaptic activity, and immunostaining.

As described below in the Examples, the present inventors revealed that using the cell culture method of the present embodiment makes the effect of suppressing the peeling of cells from the culture surface sufficiently significant. Specifically, it was revealed that, in comparison with a culture method using a cell culture container in which the culture surface is immersed in a serum culture medium, as described in Non Patent Document 3, there was little variation between wells during extracellular potential measurement using MEA and stable functional evaluation results from cells were obtained.

As described above, the cell culture container used in the cell culture method of the present embodiment includes a coat layer and the coat layer includes the layer (i-1) including gelatin or casein. Examples of gelatin include gelatin derived from cow bones, gelatin derived from cow skin, gelatin derived from pig skin, gelatin derived from donkey skin, gelatin derived from chicken skin, gelatin derived from fish, and the like. Examples of gelatin derived from fish include gelatin derived from fish scale, gelatin derived from fish skin, and the like. Among the above, gelatin derived from fish is particularly preferable due to having a low gelation temperature and being easy to handle. The above may be used alone or in a combination of two or more.

Examples of casein include casein derived from cows, casein derived from goats, casein derived from sheep, casein derived from humans, and the like. Among the above, casein derived from cows is preferable due to being abundant in milk and inexpensive. The above may be used alone or in a combination of two or more.

As described above, the cell culture container used in the cell culture method of the present embodiment includes a coat layer and the coat layer includes the layer (i-2) that includes a polycationic material. It is possible to select, as appropriate, polycationic materials depending on the purpose without being particularly limited and polyethyleneimine (PEI), polylysine (PLL or PDL), and polyornithine (PLO or PDLO) are preferable, for which there are numerous instances in cell culture. The above may be used alone or in a combination of two or more.

As described above, the cell culture container used in the cell culture method of the present embodiment includes a coat layer. The coat layer includes the layer (i) and the layer (ii) that is layered on the layer (i) and includes a cell adhesion factor, or the coat layer includes the layer (i) and the culture medium includes the cell adhesion factor.

It is possible to select, as appropriate, cell adhesion factors depending on the purpose without being particularly limited, but an extracellular matrix is preferable. The extracellular matrix, also called an extracellular base material, is a substance that serves as a scaffold for cells. Examples of the extracellular matrix include laminin, collagen, fibronectin, fibrinogen, Matrigel (registered trademark, Corning), Geltrex (Thermo Fisher Scientific, Inc.), and the like. The above may be used alone or in a combination of two or more.

Among the above, in a case where the cells to be cultured are cell aggregates including nerve cells, laminins and Matrigel (registered trademark) are preferable from the viewpoint of promoting the maturation of nerve cells.

### [Cell Culture Container Having Modified Culture Surface]

The cell culture container in one embodiment is a cell culture container having a modified culture surface. The cell culture container of the present embodiment has a coat layer layered on a surface and the coat layer includes the layer (i) that includes the layer (i-1) including gelatin or casein and the layer (i-2) that includes a polycationic material and the layer (ii) that is layered on the layer (i) and includes a cell adhesion factor, and the layer (ii) is disposed at a position at which the layer (ii) comes into contact with cells during cell culture. The culture surface refers to the part of the surface of the cell culture container to which cells adhere during cell culture. In a case where a coat layer is layered on the surface of the base material of the cell culture container, the culture surface is the surface of the coat layer.

FIGS. 2(a) and 2(b) are schematic cross-sectional diagrams of the cell culture surface portion of a cell culture container, each of which is an example of the cell culture container of the present embodiment. In the cell culture container shown in FIG. 2(a), a layer 22 including gelatin or casein, a layer 23 including a polycationic material, and a layer 24 including a cell adhesion factor are layered in this order on the surface of a base material 21.

Here, it is preferable that the base material 21 and the layer 22 are adjacent to each other and no other substances are present between the surface of the base material 21 and the layer 22. In addition, it is preferable that the layer 22 and the layer 23 are adjacent to each other and no other substances are present between the layer 22 and the layer 23. In addition, it is preferable that the layer 23 and the layer 24 are adjacent to each other and no other substances are present between the layer 23 and the layer 24.

In the cell culture container shown in FIG. 2(b), the layer 23 including a polycationic material, the layer 22 including gelatin or casein, and the layer 24 including a cell adhesion factor are layered in this order on the surface of the base material 21.

Here, it is preferable that the base material 21 and the layer 23 are adjacent to each other and no other substances are present between the surface of the base material 21 and the layer 23. In addition, it is preferable that the layer 23 and the layer 22 are adjacent to each other and no other substances are present between the layer 23 and the layer 22. In addition, it is preferable that the layer 22 and the layer 24 are adjacent to each other and no other substances are present between the layer 22 and the layer 24.

As described below in the Examples, the present inventors found that, when adherent culture of the cells is performed using a cell culture container in which a coat layer having the configuration described above is layered on the surface of the cell culture container, it is possible to significantly suppress the peeling of the cells from the culture surface even in a case where the cells are easily peelable cells.

Accordingly, the cell culture container of the present embodiment is suitable for culturing easily peelable cells. Examples of easily peelable cells include cells that are peeled from the culture surface of a cell culture container simply by the application of shocks or vibration to the cell culture container. Specific examples of easily peelable cells include cell aggregates including nerve cells, HEK293, which is a human embryonic kidney cell line, and the like.

As described above, when the peeling of cell aggregates including nerve cells from the culture surface is suppressed, it is possible to stably detect and evaluate the action potentials of nerve cells using MEA or the like. In addition, it is possible to obtain stable evaluation results by other analysis methods such as detection of changes in Ca concentration, observation of synaptic activity, and immunostaining.

As described below in Examples, the present inventors revealed that using the cell culture container of the present embodiment makes the effect of suppressing the peeling of cells from the culture surface sufficiently significant. Specifically, it was revealed that, in comparison with a cell culture container in which the culture surface is immersed in a serum culture medium, as described in Non Patent Document 3, there was little variation between wells during extracellular potential measurement using MEA and functional evaluation results from stable cells were obtained.

The cell culture container may be a cell culture container commonly used for cell culturing and examples thereof include a dish, a well plate, and the like. It is possible to select, as appropriate, the diameter of the dish, the number of wells in the well plate, and the like depending on the application. The culture surface refers to the part of the surface of the cell culture container to which cells adhere during cell culture.

In the cell culture container of the present embodiment, an electrode array may be disposed on the culture surface. That is, the cell culture container of the present embodiment may be an MEA plate. It is possible to select, as appropriate, the number of electrodes of the MEA or the like depending on the application.

Examples of the material for the base material of the cell culture container include the organic materials and inorganic materials described below. The above may be used alone or in a combination of two or more.

It is possible to select, as appropriate, the organic material depending on the purpose without being particularly limited and examples thereof include polyethylene terephthalate (PET), polystyrene (PS), polycarbonate (PC), TAC (triacetyl cellulose), polyimide (PI), nylon (Ny), low density polyethylene (LDPE), medium density polyethylene (MDPE), vinyl chloride, vinylidene chloride, polyphenylene sulfide, polyether sulfone, polyethylene naphthalate, polypropylene, polyacrylamide, acrylic materials such as urethane acrylate, cellulose, silicone-based materials such as polydimethylsiloxane (PDMS), polyvinyl alcohol (PVA), alginate metal salts such as calcium alginate, gel-like materials such as methylcellulose and agarose, and the like.

It is possible to select, as appropriate, the inorganic material depending on the purpose without being particularly limited and examples thereof include glass, ceramics, and the like.

As described above, the cell culture container of the present embodiment includes a layer including gelatin or casein. The gelatin is the same as described above and is preferably gelatin derived from fish. In addition, the casein is the same as described above. Furthermore, as described above, the cell culture container of the present embodiment includes a layer including a polycationic material. The polycationic material is the same as described above and is preferably polyethyleneimine, polylysine, or polyornithine. In addition, as described above, the cell culture container of the present embodiment includes a layer including a cell adhesion factor. The cell adhesion factors are the same as described above and laminins and Matrigel (registered trademark) are preferable.

### [Method for Producing Cell Culture Container Having Modified Culture Surface]

In one embodiment, the present invention provides a method for producing a cell culture container having a modified culture surface including a step 1 of bringing a solution including gelatin or casein into contact with a surface of the cell culture container, a step 2 of removing the solution including the gelatin or the casein, a step 3 of bringing a solution including a polycationic material into contact with the surface of the cell culture container, a step 4 of removing the solution including the polycationic material, a step 5 of bringing a solution including a cell adhesion factor into contact with the surface of the cell culture container, and a step 6 of removing the solution including the cell adhesion factor, in which the production method is performed in the above order, or in an order of the step 3, the step 4, the step 1, the step 2, the step 5, and the step 6.

It is possible to produce the cell culture container described above using the production method of the present embodiment. As described below in Examples, culturing a cell aggregate including nerve cells in a cell culture container produced by the production method of the present embodiment makes it possible to significantly suppress peeling of cells from the culture surface.

The cell culture container is the same as described above. The cell culture container may be a dish or a well plate or may be an MEA plate having an electrode array disposed on the culture surface.

In a case where the production method of the present embodiment includes the above steps in the order of the step 1, the step 2, the step 3, the step 4, the step 5, and the step 6, there may be a further step of carrying out a hydrophilic treatment on the surface of the cell culture container before the step 1. Alternatively, in a case where the production method of the present embodiment includes the above steps in the order of the step 3, the step 4, the step 1, the step 2, the step 5, and the step 6, there may be a further step of carrying out a hydrophilic treatment on the surface of the cell culture container before the step 3.

Subjecting the cell culture surface to a hydrophilic treatment facilitates the surface modification using gelatin, casein, or a polycationic material, which will be described below. The hydrophilic treatment of the cell culture surface may be carried out by methods known in the related art without being limited thereto and examples thereof include methods such as treating the cell culture surface with ethanol or pure water, or carrying out an ozone plasma treatment on the cell culture surface. A description will be given below of the hydrophilic treatment using a method for treating the cell culture surface using ethanol as an example.

The step of carrying out the hydrophilic treatment on the surface of the cell culture container may include a step (a) of bringing a solution including ethanol into contact with the surface of the cell culture container and a step (b) of removing the solution including the ethanol.

In the step (a), a solution including ethanol is brought into contact with the surface of the cell culture container. Specifically, a solution including ethanol may be added to the cell culture container, or the entire cell culture container may be immersed in a solution including ethanol.

As the solution including ethanol, an ethanol aqueous solution is preferable. The concentration of the ethanol is preferably approximately 70% by mass. The ethanol aqueous solution is not particularly limited, but it is preferably prepared from a highly pure ethanol solution and ultrapure water in order to prevent influence on the cells as much as possible.

The time for carrying out the contact with the solution including ethanol is preferably approximately 2 minutes to 10 minutes. In addition, the temperature for carrying out the contact with the solution including ethanol is preferably approximately room temperature which is 18°C to 25°C.

Subsequently, in the step (b), the solution including ethanol is removed. After removing the solution including the ethanol, it is preferable to dry the culture surface. The drying method is not particularly limited, but examples thereof include air drying in a sterile environment or the like.

Subsequently, in the step 1, a solution including gelatin or casein is brought into contact with the surface of the cell culture container. Here, the surface of the cell culture container means a surface modified by contact with a solution including ethanol. Specifically, a solution including gelatin or casein may be added to a cell culture container, or the entire cell culture container may be immersed in a solution including gelatin or casein.

The gelatin is the same as described above and is preferably gelatin derived from fish. Examples of solutions including gelatin include gelatin aqueous solutions and gelatin buffer solutions. It is possible to select, as appropriate, the materials included in the solution including gelatin depending on the purpose without being particularly limited other than not being toxic to cells, but the concentration of gelatin in the solution is preferably 0.05 to 2% by mass from the viewpoints of being able to carry out even layering on the surface of the cell culture container and of production at low cost.

In addition, the casein is the same as described above. Examples of solutions including casein include casein aqueous solutions and casein buffer solutions. It is possible to select, as appropriate, the materials included in the solution including casein depending on the purpose without being particularly limited other than not being toxic to cells, but the concentration of casein in the solution is preferably 0.01 to 2% by mass from the viewpoints of being able to carry out even layering on the surface of the cell culture container and of production at low cost.

The contact time with the solution including gelatin or casein is preferably one hour or more. In addition, the temperature for carrying out the contact with the solution including gelatin or casein is preferably lower than 40°C so as not to lose the characteristics of gelatin or casein. Specifically, for example, it is possible to allow the solution to stand at approximately 37°C for one hour to carry out the gelatin or casein layering efficiently on the surface of the cell culture container.

Subsequently, in the step 2, the solution including gelatin or casein is removed. After removing the solution including gelatin or casein, the surface of the cell culture container may be washed with water or a buffer solution. The number of washes may be from one to several times. For example, the number of washes may be 2 to 4 times, for which there are numerous instances in cell culture. Subsequently, it is preferable to dry the surface of the cell culture container. The drying method is not particularly limited, but examples include air drying in a sterile environment or the like.

Subsequently, in the step 3, a solution including a polycationic material is brought into contact with the surface of the cell culture container. Specifically, a solution including a polycationic material may be added to a cell culture container or the entire cell culture container may be immersed in a solution including a polycationic material.

The polycationic material is the same as described above and is preferably polyethyleneimine, polylysine, or polyornithine. Examples of solutions including polycationic materials include aqueous solutions of polycationic materials and buffer solutions of polycationic materials. The concentration of polycationic material in the solution is preferably 0.001 to 0.2% by mass, for which there are numerous instances in cell culture.

The time for carrying out the contact with the solution including the polycationic material is preferably one hour or more. In addition, the temperature for carrying out the contact with the solution including the polycationic material is not particularly limited. Specifically, for example, it is possible to allow the solution to stand at approximately 37°C for one hour, for which there are numerous instances in cell culture, to carry out the polycationic material layering efficiently on the surface of the cell culture container.

Subsequently, in the step 4, the solution including the polycationic material is removed. After removing the solution including the polycationic material, the number of washes may be from one to several times. For example, the number of washes may be 2 to 4 times, for which there are numerous instances in cell culture. Subsequently, it is preferable to dry the surface of the cell culture container. The drying method is not particularly limited, but examples include air drying in a sterile environment or the like.

Subsequently, in the step 5, a solution including a cell adhesion factor is brought into contact with the surface of the cell culture container. Here, the surface of the cell culture container means a surface modified by carrying out the step 1, the step 2, the step 3, and the step 4 described above. Specifically, a solution including a cell adhesion factor may be added to the surface of a cell culture container or the entire cell culture container may be immersed in a solution including a cell adhesion factor.

The cell adhesion factors are the same as described above and an extracellular matrix is preferable. Examples of solutions including cell adhesion factors include aqueous solutions of cell adhesion factors and buffer solutions of cell adhesion factors. The concentration of the cell adhesion factor in the solution is preferably 5 to 20 µg/mL for laminin or 10 to 50 µg/mL for Matrigel, for which there are numerous instances in cell culture.

The time for carrying out the contact with the solution including the cell adhesion factor is preferably one hour or more. Furthermore, the temperature for carrying out the contact with the solution including the cell adhesion factor is not particularly limited. Specifically, for example, it is possible to allow the solution to stand at approximately 37°C for one hour, for which there are numerous instances in cell culture, to carry out the cell adhesion factor layering efficiently on the surface of the cell culture container.

Subsequently, in the step 6, the solution including a cell adhesion factor is removed. After removing the solution including a cell adhesion factor, the surface of the cell culture container may be washed with water or a buffer solution. The number of washes may be from one to several times. In a case where the cell adhesion factor is Matrigel, the surface of the cell culture container may then be dried. The drying method is not particularly limited, but examples include air drying in a sterile environment or the like.

In the production method of the present embodiment, the step 1 to the step 6 described above may be carried out in this order. Alternatively, the steps described above may be carried out in order of the step 3, the step 4, the step 1, the step 2, the step 5, and the step 6. In addition, in a case of performing a hydrophilic treatment, the steps described above may be carried out in the order of the step (a), the step (b), the step 1, the step 2, the step 3, the step 4, the step 5, and the step 6. Alternatively, the steps described above may be carried out in the order of the step (a), the step (b), the step 3, the step 4, the step 1, the step 2, the step 5, and the step 6. That is, the order of carrying out contact with and removing a solution including gelatin or casein and carrying out contact with and removing a solution including a polycationic material may be reversed. As described below in the Examples, in either case, it is possible to produce a cell culture container able to significantly suppress the peeling of cells from a culture surface.

### [Cells and Culture Medium]

It is possible to select, as appropriate, the cells to be cultured in the cell culture container of the present embodiment depending on the purpose without being particularly limited other than being adherent cells and examples thereof include endothelial cells such as hepatocytes, astrocytes, Kupffer cells, vascular endothelial cells, sinusoidal endothelial cells, and corneal endothelial cells, epidermal cells such as fibroblasts, osteoblasts, osteoclasts, periodontal membrane-derived cells, and epidermal keratinocytes, epithelial cells such as tracheal epithelial cells, intestinal epithelial cells, cervical epithelial cells, and corneal epithelial cells, myocytes such as mammary gland cells, pericytes, smooth muscle cells, and cardiomyocytes, renal cells, pancreatic islet of Langerhans cells, nerve cells such as peripheral nerve cells and optic nerve cells, cartilage cells, bone cells, and the like. Among the above, nerve cells are preferable as the adherent cells.

Nerve cells may be primary cells directly collected from tissues or organs or may be obtained by subculturing these primary cells for several generations. In addition, from the viewpoint of making it easier to obtain a cell population including a large number of desired nerve cells, cells induced to differentiate from stem cells may be used. That is, the nerve cells may be stem cell-derived nerve cells.

Examples of stem cells include embryonic stem cells (ES cells), artificial pluripotent stem cells, mesenchymal stem cells, umbilical cord blood-derived stem cells, neural stem cells, and the like. Examples of artificial pluripotent stem cells include nuclear transfer embryonic stem cells (ntES cells), induced pluripotent stem cells (iPS cells), and the like. Examples of mesenchymal stem cells include bone marrow mesenchymal stem cells, adipose tissue-derived mesenchymal stem cells, and the like. Among the above, iPS cells are preferable as stem cells.

iPS cells may be derived from healthy individuals or patients with various neurological diseases. In addition, the cells may be subjected to various types of gene editing, for example, cells manipulated by gene editing to have a gene that is a cause or risk factor for various neurological diseases.

In a case where iPS cells are derived from patients with various neurological diseases, it is possible to use these cells to construct patient models of the nervous system diseases. Without being particularly limited, nervous system diseases include neurodegenerative diseases, autism, epilepsy, attention-deficit hyperactivity disorder (ADHD), schizophrenia, bipolar disorder, and the like. Examples of neurodegenerative diseases include Alzheimer's disease, Parkinson's disease, amyotrophic lateral sclerosis, and the like.

The animal species from which the nerve cells are derived are not particularly limited and examples thereof include humans, monkeys, dogs, cows, horses, sheep, pigs, rabbits, mice, rats, guinea pigs, hamsters, and the like. Among the above, humans are preferable.

In addition, one type of nerve cell may be used alone, or a mixture of two or more types of nerve cells may be used. It is possible to broadly classify nerve cells into, for example, peripheral nerves and central nerves. Examples of peripheral nerves include sensory nerve cells, motor nerve cells, and autonomic nerve cells. Examples of central nerves include interneurons and projection neurons. Examples of projection neurons include cortical neurons, hippocampal neurons, amygdala neurons, and the like. Furthermore, it is possible to broadly classify central nerve cells into excitatory neurons and inhibitory neurons.

Examples include glutamatergic neurons, which are mainly responsible for excitatory transmission in the central nervous system, and GABAergic (γ-aminobutyric acid) neurons, which are mainly responsible for inhibitory transmission. Other examples of neurons that release neuromodulators include cholinergic neurons, dopaminergic neurons, noradrenergic neurons, serotonergic neurons, histaminergic neurons, and the like.

The cells cultured in the cell culture container of the present embodiment may include astrocytes, microglia, and the like, in addition to nerve cells. Further, depending on the purpose, it is preferable that the nerve cells are mature and, for example, among Tubulin beta3, MAP2, NeuN, 160 kDa Neurofilament, 200 kDa Neurofilament, NSE, PSD93, PSD95, and the like, the expression of any marker gene is preferably positive.

It is possible to select, as appropriate, the culture medium used in the cell culture container of the present embodiment depending on the purpose without being particularly limited and examples thereof include culture media classified by composition such as natural culture media, semi-synthetic culture media, and synthetic culture media; culture media classified by shape such as semi-solid culture media, liquid culture media, and powder culture media, and the like. The above may be used alone or in a combination of two or more. In a case where the cells are derived from animals, it is possible to use any culture medium used for culturing animal cells.

Examples of the culture medium used for nerve cells include a culture medium obtained by adding necessary components to a basal medium. Examples of the basal medium include Dulbecco's Modified Eagle's Medium (DMEM), Ham's Nutrient Mixture F12, D-MEM/F12 medium, McCoy's 5A medium (McCoy's 5A medium), Eagle's Minimum Essential Medium (EMEM), αMEM medium (alphaModified Eagle's Minimum Essential Medium, αMEM), MEM medium (Minimum Essential Medium), RPMI1640 (Roswell Park Memorial Institute-1640) medium, Iscove's Modified Dulbecco's Medium (IMDM), MCDB131 medium, William's medium E, IPL41 medium, Fischer's medium, M199 medium, Hight Performance Medium 199, StemPro34 (produced by Thermo Fisher Scientific, Inc.), X-VIVO 10 (produced by Chembrex), X-VIVO 15 (produced by Chembrex), HPGM (produced by Chembrex), StemSpan H3000 (produced by Stemcell Technologies), StemSpanSFEM (produced by Stemcell Technologies), Stemline II (produced by Sigma-Aldrich), QBSF-60 (produced by Quality Biological), StemProhESCSFM (produced by Thermo Fisher Scientific, Inc.), Essentials (registered trademark) medium (produced by Thermo Fisher Scientific, Inc.), mTeSR1 or mTeSR2 medium (produced by Stemcell Technologies), ReproFF or ReproFF2 (produced by Reprocell, Inc.), PSGro hESC/iPSC medium (produced by System Biosciences, LLC.), NutriStem (registered trademark) medium (produced by Biological Industries, Inc.), CSTI-7 medium (produced by Cell Science & Technology Institute, Inc.), MesenPRO RS medium (produced by Thermo Fisher Scientific, Inc.), MF-Medium (registered trademark) mesenchymal stem cell growth medium (produced by Toyobo Co., Ltd.), Sf-900II (produced by Thermo Fisher Scientific, Inc.), Opti-Pro (produced by Thermo Fisher Scientific, Inc.), and the like. The above may be used alone or in a mixture of two or more.

In addition, examples of additives to be added to the basal medium include additives commonly used for nerve cell culture and examples thereof include Component N (Elixirgen Scientific, Inc.), Component G2 (Elixirgen Scientific, Inc.), N2 Supplement (Thermo Fisher Scientific, Inc.), iCell Neural Supplement B (CDI Inc.), iCell Nervous System Supplement, B-27 plus (Thermo Fisher Scientific, Inc.), and the like.

It is possible to select, as appropriate, other components depending on the purpose without being particularly limited and examples thereof include a culture medium, a crosslinking agent, a pH adjuster, a preservative, an antioxidant, and the like.

It is possible to select, as appropriate, the carbon dioxide concentration in the culture medium depending on the purpose without being particularly limited, but 2% or more and 5% or less is preferable and 3% or more and 4% or less is more preferable. When the carbon dioxide concentration is 2% or more and 5% or less, it is possible to suitably culture the cells.

### [Examples]

A more detailed description will be given below of the present invention with reference to Examples, but the present invention is not limited to the following Examples.

### [Example 1]

### (Preparation of Cell Culture Container of Example 1)

### «Cell Culture Container»

A96-Well Black/Clear Bottom Plate, TC Surface (96-well plate, model number "165305", Thermo Fisher Scientific, Inc.) was used.

### «70% by Mass Ethanol Aqueous Solution»

A 70% by mass ethanol solution was prepared using Ethanol (99.5) (model number "08948-25", Nacalai Tesque, Inc.) and ultrapure water.

### «Aqueous Solution Including Gelatin»

Gelatin from cold water fish skin (model number "G7041", Sigma-Aldrich) was dissolved in ultrapure water and an aqueous solution including gelatin having a gelatin derived from fish concentration of 1% by mass was prepared.

### «Aqueous Solution Including Polycationic Material»

Poly-L-ornithine solution (PLO, model number "P4957-50ML", Sigma-Aldrich) was diluted with "DPBS, no calcium, no magnesium" (DPBS, model number "14190250", Thermo Fisher Scientific, Inc.) and an aqueous solution including a polycationic material having a polyornithine concentration of 0.002% by weight was prepared.

### «Aqueous Solution Including Cell Adhesion Factor»

"Laminin Mouse Protein, Natural" (Laminin, model number "23017015", Thermo Fisher Scientific, Inc.) was diluted with DPBS and an aqueous solution including 10 µg/mL of a cell adhesion factor protein was prepared.

### <<Preparation of Cell Culture Container of Example 1>>

A 70% by mass ethanol aqueous solution was placed in a cell culture container and allowed to stand at room temperature for 5 minutes. Subsequently, the 70% by mass ethanol aqueous solution was removed from the cell culture container, which was then dried in a safety cabinet. After drying, an aqueous solution including gelatin was placed in the cell culture container and allowed to stand at 37°C for one hour. Afterwards, the aqueous solution including gelatin was removed and the container was washed once with ultrapure water and dried in a safety cabinet. After drying, an aqueous solution including a polycationic material was placed in the cell culture container and allowed to stand at 37°C for one hour. Thereafter, the aqueous solution including the polycationic material was removed and the container was washed twice with DPBS and once with ultrapure water and dried in a safety cabinet. After drying, an aqueous solution including a cell adhesion factor was placed in the cell culture container and allowed to stand at 37°C for one hour. Thereafter, the aqueous solution including the cell adhesion factor was removed and the container was washed once with DPBS to obtain a cell culture container of Example 1 having a modified culture surface.

### [Example 2]

### (Preparation of Cell Culture Container of Example 2)

### «Cell Culture Container, 70% by Mass Ethanol Aqueous Solution, Aqueous Solution Including Polycationic Material, and Aqueous Solution Including Cell Adhesion Factor»

For the cell culture container, 70% by mass ethanol aqueous solution, aqueous solution including a polycationic material, and aqueous solution including a cell adhesion factor, the same examples as in Example 1 were used.

### <<Aqueous Solution Including Casein>>

Blocker^{™} Casein in PBS (model number "37582", Thermo Fisher Scientific, Inc.) was diluted with DPBS and an aqueous solution including casein having a casein concentration of 0.1% by mass was prepared.

### <<Preparation of Cell Culture Container of Example 2»

A cell culture container of Example 2 having a modified culture surface was obtained in the same manner as in Example 1 except that an aqueous solution including casein was used instead of an aqueous solution including gelatin.

### [Comparative Example 1]

### (Preparation of Cell Culture Container of Comparative Example 1)

### «Cell Culture Container, Aqueous Solution Including Polycationic Material, and Aqueous Solution Including Cell Adhesion Factor»

For the cell culture container, aqueous solution including a polycationic material, and aqueous solution including a cell adhesion factor, the same examples as in Example 1 were used.

### <<Preparation of Cell Culture Container of Comparative Example 1»

A cell culture container of Comparative Example 1 having a modified culture surface was obtained in the same manner as in Example 1, except that the step of bringing a 70% by mass ethanol aqueous solution into contact with the culture surface of the cell culture container and the step of bringing an aqueous solution including gelatin into contact therewith were not performed.

### [Comparative Example 2]

### (Preparation of Cell Culture Container of Comparative Example 2)

### «Cell Culture Container, 70% by Mass Ethanol Aqueous Solution, Aqueous Solution Including Polycationic Material, and Aqueous Solution Including Cell Adhesion Factor»

For the cell culture container, 70% by mass ethanol aqueous solution, aqueous solution including a polycationic material, and aqueous solution including a cell adhesion factor, the same examples as in Example 1 were used.

### <<Serum Culture Medium>>

A 10% by volume fetal bovine serum and a 1% by volume antibiotic-antimycotic mixed solution (100 times concentrated) (model number "02892-54", Nacalai Tesque, Inc.) was mixed into "DMEM, low glucose, GlutaMAXtm Supplement, pyruvate" (model number "10567014", Thermo Fisher Scientific, Inc.) and a serum culture medium was prepared.

### <<Preparation of Cell Culture Container of Comparative Example 2»

A cell culture container of Comparative Example 2 having a modified culture surface was obtained in the same manner as in Example 1, except that a serum culture medium was used instead of the aqueous solution including gelatin and the condition under which the serum culture medium was brought into contact with the surface of the cell culture container was changed to 2 minutes at room temperature.

### [Experiment Example 1]

### (Verification of Peeling of Cell Aggregates in 96-Well Plate)

Nerve cells were seeded into each of 24 wells of the cell culture containers of Examples 1 and 2 and Comparative Examples 1 and 2. As the nerve cells, a cell mixture of nerve cells obtained by causing iPS cell lines derived from healthy individuals to differentiate into nerve cells (product name: "Human iPSC-derived GABAergic Neurons", Elixagen Scientific) and astrocytes (product name: "Human Primary Astrocytes", Thermo Fisher Scientific, Inc.) was used.

After culturing the nerve cells for 5 weeks, each well was observed under a microscope and the number of wells in which half or more of the culture surface area had peeled was calculated. The results are shown in Table 1 below.

**Table 1**

| Cell culture container | Example 1 | Example 2 | Comparative Example 1 | Comparative Example 2 |
|---|---|---|---|---|
| Note | Including gelatin | Including casein | Not including anything | Including serum culture medium |
| Number of peeled wells | 1 | 0 | 6 | 11 |

As a result, in comparison with the culture method using the cell culture containers of Comparative Examples 1 and 2, it was confirmed that the culture method using the cell culture containers of Examples 1 and 2 resulted in a small number of wells in which cell aggregates were peeled.

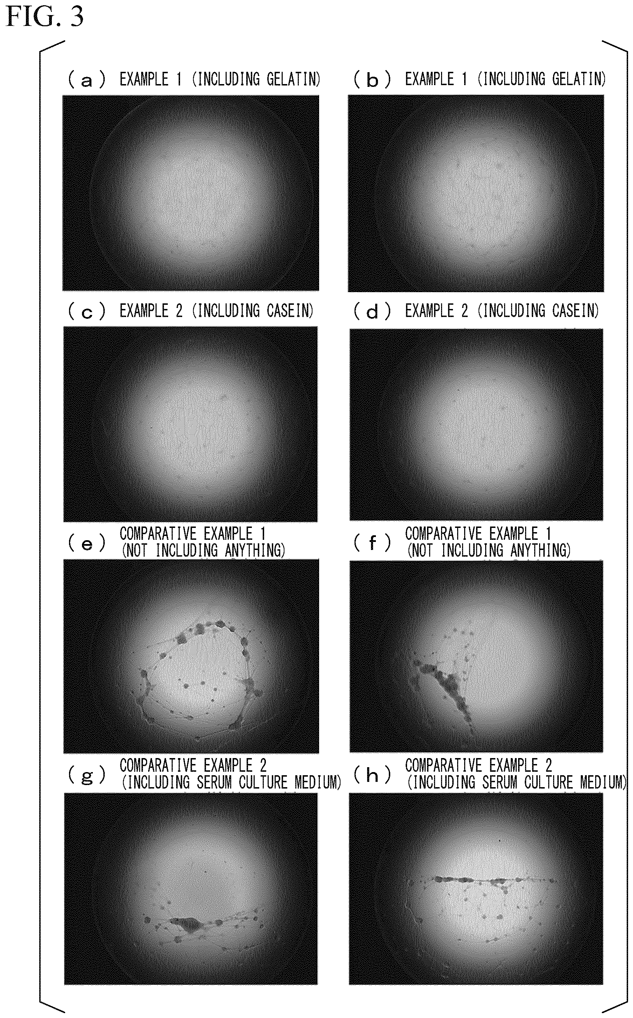

FIGS. 3(a) to 3(h) are representative microscope photographs of cell aggregates including nerve cells after 5 weeks of culturing. FIGS. 3(a) and 3(b) are microscope photographs of cell aggregates cultured in the cell culture container of Example 1, FIGS. 3(c) and 3(d) are microscope photographs of cell aggregates cultured in the cell culture container of Example 2, FIGS. 3(e) and 3(f) are microscope photographs of cell aggregates cultured in the cell culture container of Comparative Example 1, and FIGS. 3(g) and 3(h) are microscope photographs of cell aggregates cultured in the cell culture container of Comparative Example 2.

As a result, in the culture method using the cell culture containers of Comparative Examples 1 and 2, there were wells in which cell aggregates were significantly peeled, whereas in the culture method using the cell culture containers of Examples 1 and 2, it was confirmed that the cell aggregates were in an unpeeled state.

From the results in Table 1 and FIGS. 3 above, it was confirmed that the culture method using a cell culture container as in Examples 1 and 2 in which a first layer including gelatin or casein was layered on the culture surface, a second layer including a polycationic material was layered on the first layer, and a third layer including a cell adhesion factor was layered on that layer made it possible to suppress the peeling of cell aggregates from the culture surface in comparison with a culture method using the cell culture containers of Comparative Examples 1 and 2. In addition, it was confirmed that it was possible to produce the cell culture containers of Examples 1 and 2 by an inexpensive and simple method.

### [Example 3]

### (Preparation of Cell Culture Container of Example 3)

### <<70% by Mass Ethanol Aqueous Solution and Aqueous Solution Including Gelatin>>

For the 70% by mass ethanol aqueous solution and the aqueous solution including gelatin, the same examples as in Example 1 were used.

### «Cell Culture Container»

An MEA plate (model number "M768-tMEA-48W", Axion Biosystems, Inc.) having a planar microelectrode array (MEA) was used.

### <<Aqueous Solution Including Polycationic Material>>

A polyethyleneimine solution (PEI, model number "181978", Thermo Fisher Scientific, Inc.) was diluted with Pierce^{™} 20X Borate Buffer (model number "28341", Thermo Fisher Scientific, Inc.) which was diluted 20 times, and an aqueous solution including polycationic material having a concentration of 0.1% by mass of polyethyleneimine was prepared.

### <<Preparation of Cell Culture Container of Example 3»

The 70% by mass ethanol aqueous solution described above was placed in a cell culture container and allowed to stand at room temperature for 5 minutes. Subsequently, the 70% by mass ethanol aqueous solution was removed from the cell culture container, which was then dried in a safety cabinet. After drying, an aqueous solution including gelatin was placed in the cell culture container and allowed to stand at 37°C for one hour. Afterwards, the aqueous solution including gelatin was removed and the container was washed once with ultrapure water and dried in a safety cabinet. After drying, an aqueous solution including a polycationic material was placed in the cell culture container and allowed to stand at 37°C for one hour. Thereafter, the aqueous solution including the polycationic material was removed and the container was washed four times with ultrapure water and dried in a safety cabinet to obtain a cell culture container of Example 3 having a modified culture surface.

### [Comparative Example 3]

### (Preparation of Cell Culture Container of Comparative Example 3)

### «Cell Culture Container and Aqueous Solution Including Polycationic Material»

The same cell culture containers and aqueous solution including a polycationic material as in Example 3 were used.

### <<Preparation of Cell Culture Container of Comparative Example 3»

A cell culture container of Comparative Example 3 having a modified culture surface was obtained in the same manner as in Example 3, except that the step of bringing a 70% by mass ethanol aqueous solution into contact with the culture surface of the cell culture container and the step of bringing an aqueous solution including gelatin into contact therewith were not performed.

### [Comparative Example 4]

### (Preparation of Cell Culture Container of Comparative Example 4)

### «Cell Culture Container, 70% by Mass Ethanol Aqueous Solution, and Aqueous Solution Including Polycationic Material»

For the cell culture container, the 70% by mass ethanol aqueous solution, and the aqueous solution including a polycationic material, the same examples as in Example 3 were used. In addition, the same serum culture medium as in Comparative Example 2 was used.

### <<Preparation of Cell Culture Container of Comparative Example 4»

A cell culture container of Comparative Example 4 having a modified culture surface was obtained in the same manner as in Example 3, except that a serum culture medium was used instead of the aqueous solution including gelatin and the condition under which the serum culture medium was brought into contact with the surface of the cell culture container was changed to 2 minutes at room temperature.

### [Experiment Example 2]

### (Verification of Influence of Transport of MEA Plate (Axion) on Peeling of Cell Aggregates and Potential Detection)

Nerve cells were seeded into 8 wells of each of the cell culture containers of Example 3 and Comparative Examples 3 and 4. The same nerve cells as in Experiment Example 1 were used. Laminin was mixed with the cell suspension, which was then seeded in the center portion of the wells.

After culturing the nerve cells for 7 weeks, each cell culture container was placed in a container capable of maintaining a temperature and CO₂ concentration suitable for cell culture and transported by air for 16 hours. Thereafter, each well of the cell culture container was observed under a microscope and the action potentials were measured. Maestro manufactured by Axion was used to measure the action potentials. The action potentials were measured for 10 minutes in all wells.

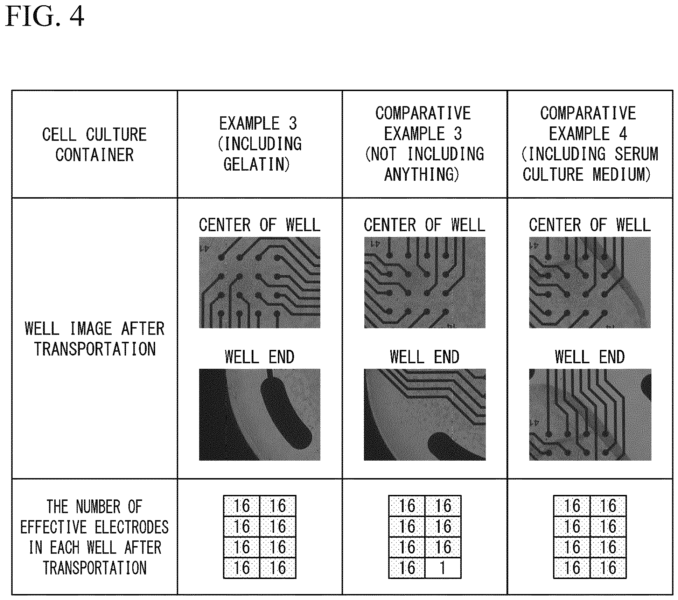

FIG. 4 is a diagram showing representative well images of each cell culture container after transportation and the number of effective electrodes in each well after transportation. The number of effective electrodes was defined as the number of electrodes able to detect the spontaneous firing of cell aggregates in each well. The maximum value of the number of effective electrodes is 16.

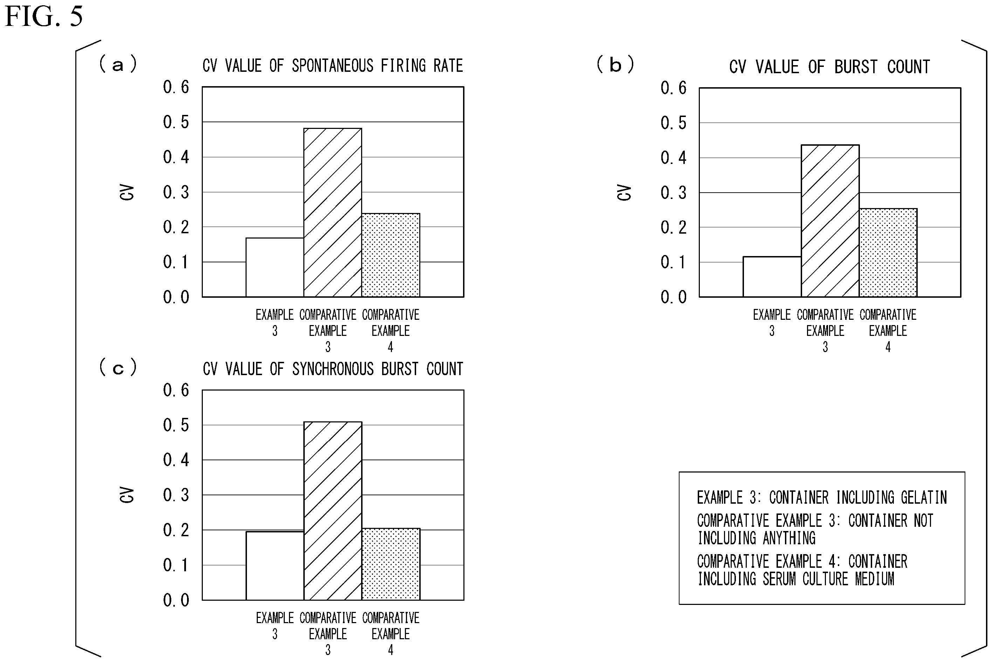

FIGS. 5(a) to 5(c) are graphs showing variations (CV values) in the spontaneous firing rate, burst count, and synchronous burst count in the culture method using each cell culture container after transportation. The CV values were calculated using Equation (1). CV value = (standard deviation value of the spontaneous firing rate, burst count, and synchronous burst count)/(average value of the spontaneous firing rate, burst count, and synchronous burst count)

From the well images after transportation in FIG. 4, in the culture method using the cell culture container of Comparative Example 4, wells in which the edges of cell aggregates were peeled were confirmed. On the other hand, in the culture method using the cell culture container of Example 3 and the cell culture container of Comparative Example 3, wells with peeled cell aggregates were not observed.

From the number of effective electrodes in FIG. 4, there was a well having a number of effective electrodes of 1 in the culture method using the cell culture container of Comparative Example 3. It was inferred that minute peeling of cells from the culture surface occurred to a degree which was not confirmable by microscopic observation. On the other hand, in the culture method using the cell culture container of Example 3 and the cell culture container of Comparative Example 4, the maximum value of the number of effective electrodes was 16 in all wells.

From FIG. 5, it was confirmed that, in all of spontaneous firing rates, burst count, and synchronous burst count, the culture method using the cell culture container of Example 3 had little variation in comparison with the culture method using the cell culture container of Comparative Example 3 and the cell culture container of Comparative Example 4. The results summarized above are shown in Table 2 below.

**Table 2**

| Cell culture container | Example 3 | Comparative Example 3 | Comparative Example 4 |
|---|---|---|---|
| Reference | Including gelatin | Not including anything | Including serum culture medium |
| Presence or absence of peeling after transportation | None | None | Present |
| Number of effective electrodes after transportation | 16 in all 8 wells | 1 in 1 well | 16 in all 8 wells |
| Variation in each action potential after transportation | Smallest overall | Largest overall | Overall, larger than the variation of Example 3 and smaller than the variation of Comparative Example 3 |

From Table 2 above, it was confirmed that, even when subjected to an external environmental load due to long-time transportation, the culture method having, as in Example 3, a first layer including gelatin layered on the culture surface, a second layer including a polycationic material layered on the first layer, and a liquid (culture medium) including a cell adhesion factor layered on that layer made it possible to suppress the peeling of cell aggregates from the culture surface and obtain stable functional evaluation results in comparison with the culture method using the cell culture containers of Comparative Examples 3 and 4.

### [Comparative Example 5]

### (Preparation of Cell Culture Container of Comparative Example 5)

### «Cell Culture Container, 70% by Mass Ethanol Aqueous Solution, and Aqueous Solution Including Polycationic Material»

For the cell culture container, 70% by mass ethanol aqueous solution, and aqueous solution including a polycationic material, the same examples as in Example 3 were used.

### <<Aqueous Solution Including Albumin>>

Blocker^{™} BSA (10X) in PBS (model number "37525", Thermo Fisher Scientific, Inc.) was diluted with DPBS and an aqueous solution including albumin having an albumin concentration of 3% by mass was prepared.

### <<Preparation of Cell Culture Container of Comparative Example 5>>

A cell culture container of Comparative Example 5 having a modified culture surface was obtained in the same manner as in Example 3 except that an aqueous solution including albumin described above was used instead of an aqueous solution including gelatin.

### [Experiment Example 3]

### (Verification of Influence of Allowing MEA Plate (Axion) to Stand at Room Temperature on Potential Detection of Cell Aggregates)

Nerve cells were seeded into 5 wells of each of the cell culture containers of Example 3 and Comparative Examples 3 to 5. The same nerve cells as in Experiment Example 1 were used. Laminin was mixed with the cell suspension, which was then seeded in the center of the wells.

After culturing the nerve cells for 5 weeks, each cell culture container was allowed to stand overnight in a room temperature environment and then the action potentials were measured. Maestro manufactured by Axion was used to measure the action potentials. The action potentials were measured for 10 minutes in all wells.

Table 3 below shows the average value for the effective electrodes in the culture method using each cell culture container after being allowed to stand overnight in a room temperature environment. The maximum value of the number of effective electrodes is 16.

**Table 3**

| Cell culture container | Example 3 | Comparative Example 3 | Comparative Example 4 | Comparative Example 5 |
|---|---|---|---|---|
| Reference | Including gelatin | Not including anything | Including serum culture medium | Including albumin |
| Average value of the number of effective electrodes after being left to stand at room temperature | 15.8 | 13 | 15 | 14 |

From Table 3 above, it was confirmed that the culture method using the cell culture container of Example 3 had a large average number of effective electrodes in comparison with the culture method using the cell culture containers of Comparative Examples 3 to 5 and that the effect of peeling of the cell aggregates was the smallest.

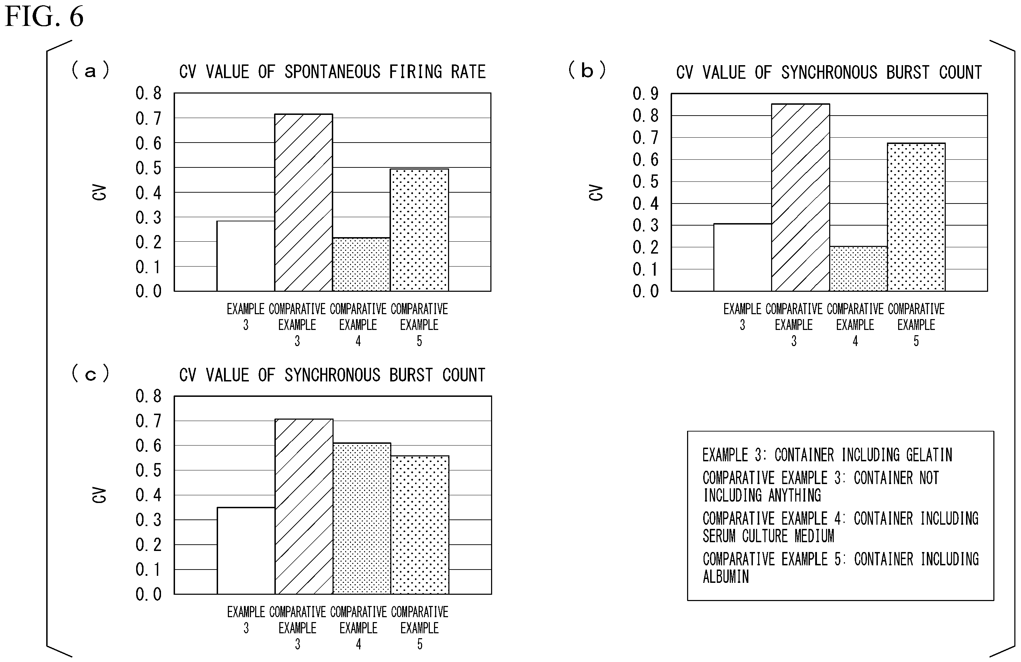

FIG. 6(a) to 6(c) are graphs showing the variations (CV values) in the spontaneous firing rate, burst count, and synchronous burst count in the culture method using each cell culture container after being allowed to stand overnight in a room temperature environment. The CV values were calculated using Equation (1).

From FIG. 6, it was confirmed that the culture method using the cell culture container in Example 3 had little variation in the synchronous burst count in comparison with the culture method using the cell culture containers of Comparative Examples 3 to 5 and that the variation in the spontaneous firing rate and the burst count was a similar level to the culture method using the cell culture container of Comparative Example 4, which had the littlest variation.

From Table 3 and FIG. 6 above, it was confirmed that, even when subjected to the external environmental load of being allowed to stand overnight in a room temperature environment, the culture method having, as in Example 3, a first layer including gelatin layered on the culture surface, a second layer including a polycationic material layered on the first layer, and a liquid (culture medium) including a cell adhesion factor layered on that layer made it possible to suppress the peeling of cell aggregates from the culture surface and obtain stable functional evaluation results in comparison with the culture method using the cell culture containers of Comparative Examples 3 to 5.

### [Example 4]

### (Preparation of Cell Culture Container of Example 4)

### <<70% by Mass Ethanol Aqueous Solution, Aqueous Solution Including Gelatin, Aqueous Solution Including Polycationic Material, and Aqueous Solution Including Cell Adhesion Factor>>

For the 70% by mass ethanol aqueous solution, aqueous solution including gelatin, and aqueous solution including polycationic material, the same examples as in Example 1 were used.

### «Cell Culture Container»

An MEA plate (model number "MaxTwo", MaxWell Biosystems) having a CMOS type planar microelectrode array (MEA) was used.

### <<Aqueous Solution Including Cell Adhesion Factor>>

Laminin was diluted with DPBS and an aqueous solution including 80 µg/mL of a cell adhesion factor was prepared.

### <<Preparation of Cell Culture Container of Example 4»

The above 70% by mass ethanol aqueous solution was placed in a cell culture container and allowed to stand at room temperature for 30 minutes. Subsequently, the 70% by mass ethanol aqueous solution was removed from the cell culture container, which was then dried in a safety cabinet. After drying, an aqueous solution including gelatin was placed in the cell culture container and allowed to stand at 37°C for one hour. Afterwards, the aqueous solution including gelatin was removed and the container was washed once with ultrapure water and dried in a safety cabinet. After drying, an aqueous solution including a polycationic material was placed in the cell culture container and allowed to stand at 37°C for 2 hours. Thereafter, the aqueous solution including the polycationic material was removed and the container was washed twice with DPBS and once with ultrapure water and dried in a safety cabinet. After drying, an aqueous solution including a cell adhesion factor was placed in the cell culture container and allowed to stand at 37°C for 30 minutes to obtain a cell culture container of Example 4 having a modified culture surface.

### [Comparative Example 6]

### (Preparation of Cell Culture Container of Comparative Example 6)

### «Cell Culture Container, 70% by Mass Ethanol Aqueous Solution, Aqueous Solution Including Polycationic Material, and Aqueous Solution Including Cell Adhesion Factor»

For the cell culture container, 70% by mass ethanol aqueous solution, aqueous solution including a polycationic material, and aqueous solution including a cell adhesion factor, the same examples as in Example 4 were used.

### <<Preparation of Cell Culture Container of Comparative Example 6»

A cell culture container of Comparative Example 6 having a modified culture surface was obtained in the same manner as Example 4, except that the step of bringing the aqueous solution including gelatin into contact with the surface of the cell culture container was not performed.

### [Experiment Example 4]

### (Verification of Peeling of Cell Aggregates Using MEA Plate (MaxWell))

Nerve cells were seeded in the wells of the cell culture containers of Example 4 and Comparative Example 6. The same nerve cells as in Experiment Example 1 were used. Laminin was mixed with the cell suspension, which was then seeded in the center of the wells.

After culturing the nerve cells for 33 days, each well was observed under a microscope and action potentials were measured. AMaxTwo system manufactured by MaxWell Biosystems was used to measure action potentials.

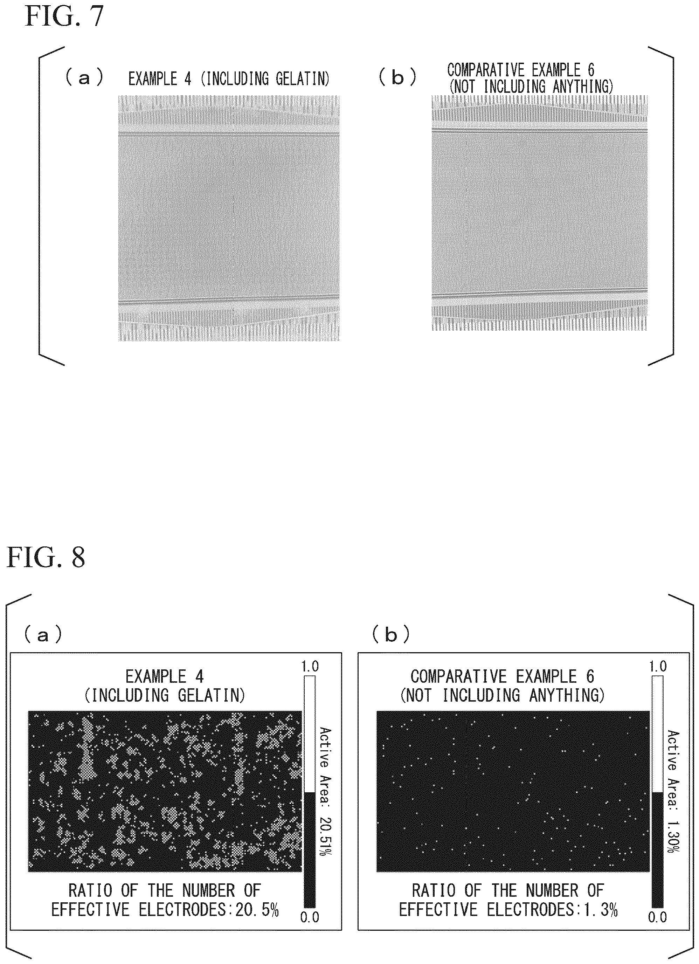

FIGS. 7(a) and 7(b) show images of the wells in each cell culture container. In addition, FIGS. 8(a) and 8(b) show mapping images of effective electrodes in the wells of each cell culture container and the ratios of the number of effective electrodes.

From FIG. 7, it was confirmed that, after 33 days of culturing, the cell aggregates were almost all peeled and lost in the culture method using the cell culture container of Comparative Example 6, whereas the cell aggregates remained without peeling in the culture method using the cell culture container of Example 4. In addition, from FIG. 8, it was confirmed that the ratio of the number of effective electrodes in the culture method using the cell culture container of Example 4 was larger than the ratio of the number of effective electrodes in the culture method using the cell culture container of Comparative Example 6 and it was possible to detect many action potentials.

From FIGS. 7 and 8, it was confirmed that, even in a CMOS type MEA plate, the culture method having, as in Example 4, a first layer including gelatin or casein layered on the culture surface, a second layer including a polycationic material layered on the first layer, and a liquid (culture medium) including a cell adhesion factor layered on that layer made it possible to suppress the peeling of cell aggregates from the culture surface and obtain stable functional evaluation results in comparison with the culture method using the cell culture container of Comparative Example 6.

### [Example 5]

### (Preparation of Cell Culture Container of Example 5)

### <<70% by Mass Ethanol Aqueous Solution, Aqueous Solution Including Gelatin, and Aqueous Solution Including Polycationic Material>>

For the 70% by mass ethanol aqueous solution, aqueous solution including gelatin, and aqueous solution including a polycationic material, the same examples as in Example 3 were used.

### «Cell Culture Container»

An MEA dish (model number "MED-R5155", AlphaMed Scientific) having a dish-shaped planar microelectrode array (MEA) was used.

### <<Preparation of Cell Culture Container of Example 5»

A cell culture container of Example 5 having a modified culture surface was obtained in the same manner as in Example 3 except that the cell culture container was different.

### [Comparative Example 7]

### (Preparation of Cell Culture Container of Comparative Example 7)

### «Cell Culture Container, 70% by Mass Ethanol Aqueous Solution, and Aqueous Solution Including Polycationic Material»

For the cell culture container, 70% by mass ethanol aqueous solution, and aqueous solution including polycationic material, the same examples as in Example 5 were used.

### <<Preparation of Cell Culture Container of Comparative Example 7»

A cell culture container of Comparative Example 7 having a modified culture surface was obtained in the same manner as in Example 5, except that the step of bringing an aqueous solution including gelatin into contact with the surface of the cell culture container was not performed.

### [Experiment Example 5]

### (Verification of Peeling of Cell Aggregates Using MEADish (Alpha Med Scientific))

Nerve cells were seeded in the cell culture containers of Example 5 and Comparative Example 7. The same nerve cells as in Experiment Example 1 were used. Laminin was mixed with the cell suspension, which was then seeded in the center of the wells.

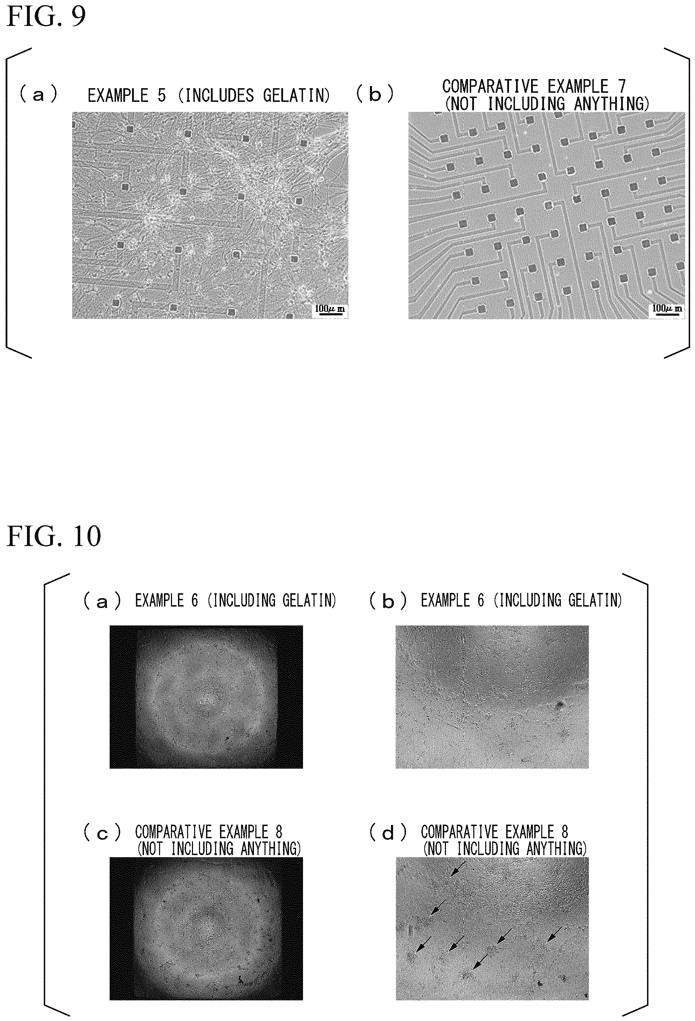

After culturing the nerve cells for 34 days, each dish was observed under a microscope. Images of each dish are shown in FIGS. 9(a) and 9(b).

From FIG. 9, it was confirmed that, after 34 days of culturing, cell aggregates were almost all peeled and lost in the culture method using the cell culture container of Comparative Example 7, whereas the cell aggregates remained without peeling in the culture method using the cell culture container of Example 5.

From FIG. 9, it was confirmed that, even in the MEA dish, the culture method having, as in Example 5, a first layer including gelatin layered on the culture surface, a second layer including a polycationic material layered on the first layer, and a liquid (culture medium) including a cell adhesion factor layered on that layer made it possible to suppress the peeling of cell aggregates from the culture surface in comparison with the culture method using the cell culture container of Comparative Example 7.

### [Example 6]

### (Preparation of Cell Culture Container of Example 6)

### «Cell Culture Container»

A 384 Well Black Plate, Optically Clear Polymer Bottom (384 well plate, model number "142761", Thermo Fisher Scientific, Inc.) was used.

### <<70% by Mass Ethanol Aqueous Solution, Aqueous Solution Including Gelatin, Aqueous Solution Including Polycationic Material, and Aqueous Solution Including Cell Adhesion Factor>>

For the 70% by mass ethanol aqueous solution, aqueous solution including gelatin, aqueous solution including a polycationic material, and aqueous solution including a cell adhesion factor, the same examples as in Example 1 were used.

### <<Preparation of Cell Culture Container of Example 6»

A cell culture container of Example 6 having a modified culture surface was obtained in the same manner as in Example 1 except that the cell culture container was different.

### [Comparative Example 8]

### (Preparation of Cell Culture Container of Comparative Example 8)

### «Cell Culture Container, Aqueous Solution including Polycationic Material, and Aqueous Solution Including Cell Adhesion Factor»

For the cell culture container, aqueous solution including a polycationic material, and aqueous solution including a cell adhesion factor, the same examples as in Example 6 were used.

### <<Preparation of Cell Culture Container of Comparative Example 8>>

A cell culture container of Comparative Example 8 having a modified culture surface was obtained in the same manner as in Example 6, except that the step of bringing an aqueous solution including gelatin into contact with the culture surface of the cell culture container was not performed.

### [Experiment Example 6]

### (Verification of Suppression of Agglomeration of Cell Aggregates in 384-Well Plate)

Nerve cells were seeded into 24 wells of each of the cell culture containers of Example 6 and Comparative Example 8. As the nerve cells, a cell mixture of nerve cells obtained by causing iPS cell lines derived from healthy individuals to differentiate into nerve cells (product name: "Human iPSC-derived GABAergic Neurons", Elixagen Scientific) and astrocytes (product name: "Human Primary Astrocytes", Thermo Fisher Scientific, Inc.) was used.

After the culturing the nerve cells for 32 days, each well was observed under a microscope. FIGS. 10(a) and 10(c) show images of each well observed with an x4 objective lens and (b) and (d) show images of each well observed with an x10 objective lens.

From FIG. 10, it was confirmed that, after 32 days of culturing, in the culture method using the cell culture container of Comparative Example 8, many areas were observed where cells were locally agglomerated as shown by the arrows in FIG. 10(d), whereas, in the culture method using the cell culture container of Example 6, no local agglomeration of cells was observed and the cells were cultured uniformly within the wells.

From FIG. 10, it was confirmed that, even in a 384-well plate, the culture method using, as in Example 6, a cell culture container in which a first layer including gelatin was layered on the culture surface, a second layer including a polycationic material was layered on the first layer, and a third layer including a cell adhesion factor was layered on that layer made it possible to suppress local cell agglomeration and achieve uniform culture within the wells in comparison with the culture method using the cell culture container of Comparative Example 7. In addition, it was confirmed that it was possible to produce the cell culture container of Example 6 by an inexpensive and simple method.

### [Example 7]

### (Preparation of Cell Culture Container of Example 7)

### «Cell Culture Container, 70% by Mass Ethanol Aqueous Solution, Aqueous Solution Including Gelatin, Aqueous Solution Including Polycationic Material, and Aqueous Solution Including Cell Adhesion Factor»

For the cell culture container, 70% by mass ethanol aqueous solution, aqueous solution including gelatin, aqueous solution including a polycationic material, and aqueous solution including a cell adhesion factor, the same examples as in Example 6 were used.

### <<Preparation of Cell Culture Container of Example 7»

A cell culture container of Example 7 having a modified culture surface was obtained in the same manner as in Example 1 except that the order of the step of placing an aqueous solution including gelatin in a cell culture container, allowing the container to stand at 37°C for one hour, removing the aqueous solution including gelatin, washing once with ultrapure water, and drying in a safety cabinet and the step of placing an aqueous solution including a polycationic material in a cell culture container, allowing the container to stand at 37°C for one hour, removing the aqueous solution including a polycationic material, washing twice with DPBS and once with ultrapure water, and drying in a safety cabinet, was reversed.

### [Comparative Example 9]

### (Preparation of Cell Culture Container of Comparative Example 9)

### «Cell Culture Container, 70% by Mass Ethanol Aqueous Solution, Aqueous Solution Including Gelatin, Aqueous Solution Including Polycationic Material, and Aqueous Solution Including Cell Adhesion Factor»

For the cell culture container, 70% by mass ethanol aqueous solution, aqueous solution including gelatin, aqueous solution including a polycationic material, and aqueous solution including a cell adhesion factor, the same examples as in Example 6 were used.

### <<Preparation of Cell Culture Container of Comparative Example 9»

A 70% by mass ethanol aqueous solution was placed in a cell culture container and allowed to stand at room temperature for 5 minutes. Subsequently, the 70% by mass ethanol aqueous solution was removed from the cell culture container, which was then dried in a safety cabinet. After drying, an aqueous solution including gelatin was placed in the cell culture container and allowed to stand at 37°C for one hour. Afterwards, the aqueous solution including gelatin was removed and the container was washed once with ultrapure water and dried in a safety cabinet. After drying, an aqueous solution including a cell adhesion factor was placed in the cell culture container and allowed to stand at 37°C for one hour. Thereafter, the aqueous solution including the cell adhesion factor was removed and the container was washed once with DPBS. An aqueous solution including a polycationic material was placed in the cell culture container and allowed to stand at 37°C for one hour. Thereafter, the aqueous solution including the polycationic material was removed and the container was washed twice with DPBS and once with ultrapure water to obtain a cell culture container of Comparative Example 9 having a modified culture surface.

### [Comparative Example 10]

### (Preparation of Cell Culture Container of Comparative Example 10)

### «Cell Culture Container, 70% by Mass Ethanol Aqueous Solution, Aqueous Solution Including Gelatin, Aqueous Solution Including Polycationic Material, and Aqueous Solution Including Cell Adhesion Factor»

For the cell culture container, 70% by mass ethanol aqueous solution, aqueous solution including gelatin, aqueous solution including a polycationic material, and aqueous solution including a cell adhesion factor, the same examples as in Example 6 were used.

### <<Preparation of Cell Culture Container of Comparative Example 10»

A 70% by mass ethanol aqueous solution was placed in a cell culture container and allowed to stand at room temperature for 5 minutes. Subsequently, the 70% by mass ethanol aqueous solution was removed from the cell culture container, which was then dried in a safety cabinet. After drying, an aqueous solution including a polycationic material was placed in the cell culture container and allowed to stand at 37°C for one hour. Thereafter, the aqueous solution including the polycationic material was removed and the container was washed twice with DPBS and once with ultrapure water and dried in a safety cabinet. After drying, an aqueous solution including a cell adhesion factor was placed in the cell culture container and allowed to stand at 37°C for one hour. Thereafter, the aqueous solution including the cell adhesion factor was removed and the container was washed once with DPBS. An aqueous solution including gelatin was placed in the cell culture container and allowed to stand at 37°C for one hour. Thereafter, the aqueous solution including gelatin was removed and the container was washed once with ultrapure water to obtain a cell culture container of Comparative Example 10 having a modified culture surface.

### [Comparative Example 11]

### (Preparation of Cell Culture Container of Example 11)

### «Cell Culture Container, 70% by Mass Ethanol Aqueous Solution, Aqueous Solution Including Gelatin, Aqueous Solution Including Polycationic Material, and Aqueous Solution Including Cell Adhesion Factor»

For the cell culture container, 70% by mass ethanol aqueous solution, aqueous solution including gelatin, aqueous solution including a polycationic material, and aqueous solution including a cell adhesion factor, the same examples as in Example 6 were used.

### <<Preparation of Cell Culture Container of Comparative Example 11»

A 70% by mass ethanol aqueous solution was placed in a cell culture container and allowed to stand at room temperature for 5 minutes. Subsequently, the 70% by mass ethanol aqueous solution was removed from the cell culture container, which was then dried in a safety cabinet. After drying, an aqueous solution including a cell adhesion factor was placed in the cell culture container and allowed to stand at 37°C for one hour. Thereafter, the aqueous solution including the cell adhesion factor was removed and the container was washed once with DPBS. An aqueous solution including a polycationic material was placed in the cell culture container and allowed to stand at 37°C for one hour. Thereafter, the aqueous solution including the polycationic material was removed and the container was washed twice with DPBS and once with ultrapure water. An aqueous solution including gelatin was placed in the cell culture container and allowed to stand at 37°C for one hour. Thereafter, the aqueous solution including gelatin was removed and the container was washed once with ultrapure water to obtain a cell culture container of Comparative Example 11 having a modified culture surface.

### [Comparative Example 12]

### (Preparation of Cell Culture Container of Comparative Example 12)

### «Cell Culture Container, 70% by Mass Ethanol Aqueous Solution, Aqueous Solution Including Gelatin, Aqueous Solution Including Polycationic Material, and Aqueous Solution Including Cell Adhesion Factor»

For the cell culture container, 70% by mass ethanol aqueous solution, aqueous solution including gelatin, aqueous solution including a polycationic material, and aqueous solution including a cell adhesion factor, the same examples as in Example 6 were used.

### <<Preparation of Cell Culture Container of Comparative Example 12»

A cell culture container of Comparative Example 12 having a modified culture surface was obtained in the same manner as in Comparative Example 11 except that the order of the step of placing an aqueous solution including gelatin in a cell culture container, allowing the container to stand at 37°C for one hour, removing the aqueous solution including gelatin, and washing once with ultrapure water, and the step of placing an aqueous solution including a polycationic material in a cell culture container, allowing the container to stand at 37°C for one hour, removing the aqueous solution including a polycationic material, and washing twice with DPBS and once with ultrapure water, was reversed.

### [Comparative Example 13]

### (Preparation of Cell Culture Container of Comparative Example 13)

### «Cell Culture Container, 70% by Mass Ethanol Aqueous Solution, Aqueous Solution Including Polycationic Material, and Aqueous Solution Including Cell Adhesion Factor»

For the cell culture container, 70% by mass ethanol aqueous solution, aqueous solution including a polycationic material, and aqueous solution including a cell adhesion factor, the same examples as in Example 6 were used.

### <<Preparation of Cell Culture Container of Comparative Example 13»

A cell culture container of Comparative Example 13 having a modified culture surface was obtained in the same manner as in Comparative Example 11 except that the step of placing an aqueous solution including gelatin in a cell culture container, allowing the container to stand at 37°C for one hour, removing the aqueous solution including gelatin, and washing once with ultrapure water was left out.

### [Comparative Example 14]

### (Preparation of Cell Culture Container of Comparative Example 14)

### «Cell Culture Container, 70% by Mass Ethanol Aqueous Solution, Aqueous Solution Including Gelatin, and Aqueous Solution Including Cell Adhesion Factor»

For the cell culture container, 70% by mass ethanol aqueous solution, aqueous solution including gelatin, and aqueous solution including a cell adhesion factor, the same examples as in Example 6 were used.

### <<Preparation of Cell Culture Container of Comparative Example 14»

A cell culture container of Comparative Example 14 having a modified culture surface was obtained in the same manner as in Comparative Example 9 except that the step of placing an aqueous solution including a polycationic material in a cell culture container, allowing the container to stand at 37°C for one hour, removing the aqueous solution including a polycationic material, and washing twice with DPBS and once with ultrapure water was left out.

### [Comparative Example 15]

### (Preparation of Cell Culture Container of Comparative Example 15)

### «Cell Culture Container, 70% by Mass Ethanol Aqueous Solution, Aqueous Solution Including Gelatin, and Aqueous Solution Including Cell Adhesion Factor»

For the cell culture container, 70% by mass ethanol aqueous solution, aqueous solution including gelatin, and aqueous solution including a cell adhesion factor, the same examples as in Example 6 were used.

### <<Preparation of Cell Culture Container of Comparative Example 15»

A cell culture container of Comparative Example 15 having a modified culture surface was obtained in the same manner as in Comparative Example 12 except that the step of placing an aqueous solution including a polycationic material in a cell culture container, allowing the container to stand at 37°C for one hour, removing the aqueous solution including a polycationic material, and washing twice with DPBS and once with ultrapure water was left out.

### [Comparative Example 16]

### (Preparation of Cell Culture Container of Comparative Example 16)

### «Cell Culture Container, 70% by mass Ethanol Aqueous Solution, and Aqueous Solution Including Cell Adhesion Factor»

For the cell culture container, 70% by mass ethanol aqueous solution, and aqueous solution including a cell adhesion factor, the same examples as in Example 6 were used.

### <<Preparation of Cell Culture Container of Comparative Example 16»

A cell culture container of Comparative Example 16 having a modified culture surface was obtained in the same manner as in Comparative Example 15 except that the step of placing an aqueous solution including gelatin in a cell culture container, allowing the container to stand at 37°C for one hour, removing the aqueous solution including gelatin, and washing once with ultrapure water was left out.

### [Experiment Example 7]

### (Verification of Suppression of Agglomeration and Peeling of Cell Aggregates in a 384-Well Plate)

Nerve cells were seeded into 6 wells of each of the cell culture containers of Examples 6 and 7 and Comparative Examples 8 to 16. As nerve cells, nerve cells differentiated from iPS cell lines derived from healthy individuals (product name: "Human iPSC-derived Excitatory Neurons", Elixagen Scientific) were used alone.

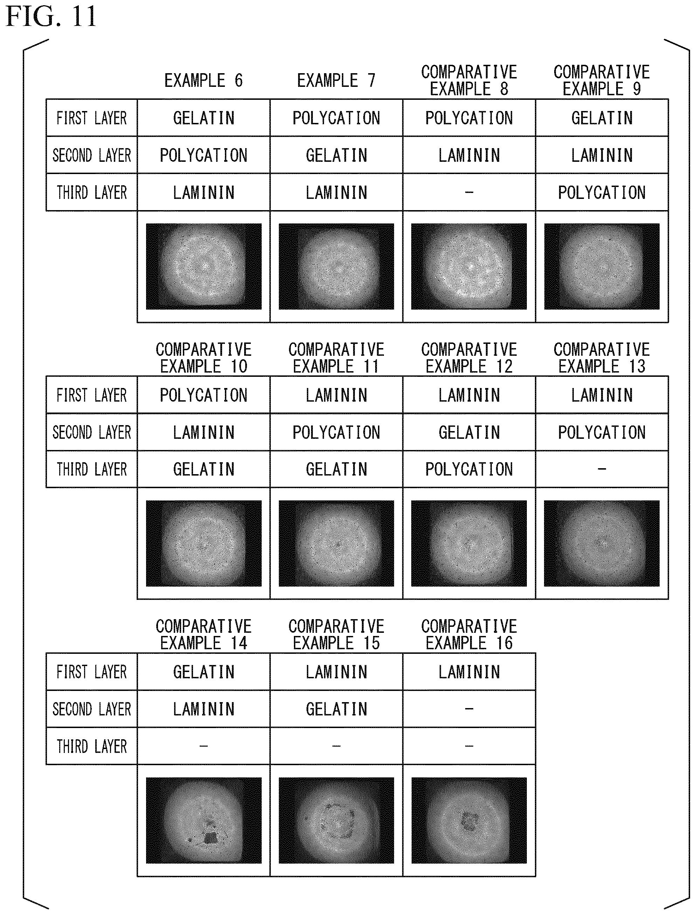

After culturing the nerve cells for 12 days, each well was observed under a microscope. FIG. 11 shows representative images of the wells of each cell culture container.

From FIG. 11, significant cell agglomeration was observed in the culture method using the cell culture containers of Comparative Examples 14 to 16 after 12 days of culturing. On the other hand, in the culture methods using the cell culture containers of Examples 6 and 7 and Comparative Examples 8 to 13, no significant cell agglomeration was observed. From FIG. 11, it was confirmed that the culture method using a cell culture container in which the layer including the polycationic material was not modified generated significant cell agglomeration.

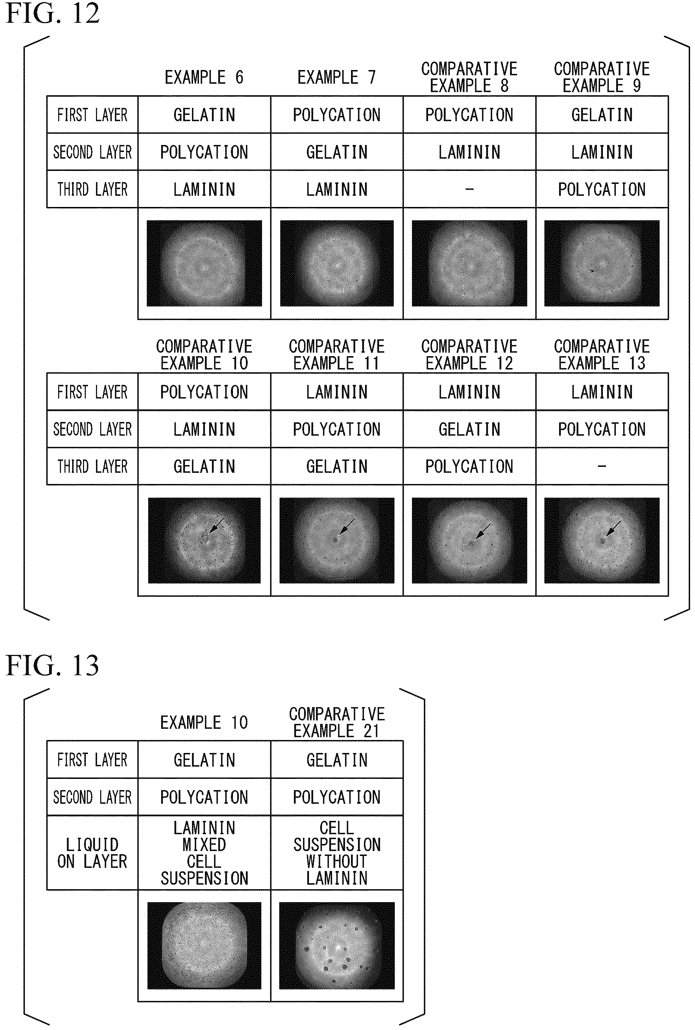

Further, in Examples 6 and 7 and Comparative Examples 8 to 13, the culturing was continued and each well was observed under a microscope after culturing for 19 days. FIG. 12 shows representative images of the wells of each cell culture container.

From FIG. 12, after 19 days of culturing, in the culture method using the cell culture containers of Comparative Examples 10 to 13, agglomeration of cells in the center portion of the well as shown by the arrow in FIG. 12 was observed. On the other hand, in the culture methods using the cell culture containers of Examples 6 and 7 and Comparative Examples 8 and 9, no agglomeration of cells in the center portion of the well was observed. From FIG. 12, it was confirmed that the culture method using a cell culture container in which the outermost layer was not modified with a layer including a cell adhesion factor had a high ratio of cell agglomeration in the center portion of the wells.

Furthermore, in Examples 6 and 7 and Comparative Examples 8 and 9, the culturing was continued for 25 days, the entire volume of the culture medium was replaced, then, each well was observed with a microscope, and the number of wells where a quarter or more of the culture surface area was peeled was calculated. The results are shown in the table below.

**Table 4**

| Cell culture container | Example 6 | Example 7 | Comparative Example 8 | Comparative Example 9 |
|---|---|---|---|---|
| First layer | Gelatin | Polycation | Polycation | Gelatin |
| Second layer | Polycation | Gelatin | Laminin | Laminin |
| Third layer | Laminin | Laminin | - | Polycation |
| Presence or absence of peeling after replacing entire volume of culture medium | 1 in all 6 wells | 1 in all 6 wells | 3 in all 6 wells | 3 in all 6 wells |

From Table 4, it was confirmed that, in comparison with the culture method using the cell culture containers of Comparative Examples 8 and 9, the culture method using the cell culture containers of Examples 6 and 7 has a low number of wells where the cell aggregates were peeled.

From the results shown in FIGS. 11 and 12 and Table 4 above, it was confirmed that the culture method using a cell culture container as in Examples 6 and 7 in which a layer including gelatin or casein and a layer including a polycationic material are layered on the culture surface, and a layer including a cell adhesion factor was layered on that layered layer made it possible to suppress the agglomeration and peeling of cell aggregates from the culture surface in comparison with the culture method using the cell culture containers of Comparative Examples 8 to 16. In addition, it was confirmed that it was also possible to produce the cell culture containers of Examples 6 and 7 by an inexpensive and simple method.

### [Example 8]

### (Preparation of Cell Culture Container of Example 8)

### «Cell Culture Container»

A Costar (R) 24-well transparent cell culture treated multi-well plate (24-well plate, model number "3526", Corning Inc.) was used.

### <<70% by Mass Ethanol Aqueous Solution, Aqueous Solution Including Gelatin, Aqueous Solution Including Polycationic Material, and Aqueous Solution Including Cell Adhesion Factor>>

For the 70% by mass ethanol aqueous solution, aqueous solution including gelatin, aqueous solution including a polycationic material, and aqueous solution including a cell adhesion factor, the same examples as in Example 1 were used.

### <<Preparation of Cell Culture Container of Example 8>>

A cell culture container of Example 8 having a modified culture surface was obtained in the same manner as in Example 1 except that the cell culture container was different.

### [Comparative Example 17]

### (Preparation of Cell Culture Container of Comparative Example 17)

### «Cell Culture Container, Aqueous Solution Including Polycationic Material, and Aqueous Solution Including Cell Adhesion Factor»

For the cell culture container, aqueous solution including a polycationic material, and aqueous solution including a cell adhesion factor, the same examples as in Example 8 were used.

### <<Preparation of Cell Culture Container of Comparative Example 17»

A cell culture container of Comparative Example 17 having a modified culture surface was obtained in the same manner as in Example 8, except that the step of bringing an aqueous solution including gelatin into contact with the culture surface of the cell culture container was not performed.

### [Comparative Example 18]

### «Cell Culture Container»

The same cell culture container as in Example 8 was used. No modification was made to the culture surface.

### [Experiment Example 8]

### (Verification of Peeling of Cell Aggregates other than Nerve Cells)

HEK293 cells were seeded into 8 wells of each of the cell culture containers of Example 8 and Comparative Examples 16 and 17.

After culturing the HEK293 cells for 4 days, the culture medium was entirely replaced, pipetting was further performed three times, and then each well was observed under a microscope to evaluate the peeling state. The results are shown in Table 5 below.

Furthermore, the entire multi-well plate was vibrated at 2.89G for 8 minutes. A vortex mixer Vortex-Genie2 (Kenis, Ltd.) was used for the vibration. Thereafter, each well was observed under a microscope to evaluate the peeling state. The results are shown in Table 5 below.

**Table 5**

| | Example 8 (including gelatin) | Comparative Example 17 (not including gelatin) | Comparative Example 18 (not modified) |
|---|---|---|---|
| After replacing entire volume of culture medium and pipetting three times | All 8 wells, no peeling | All 8 wells, no peeling | All 8 wells were approximately half peeled |
| After vibration load | All 8 wells, no peeling | In all 8 wells, 3 wells were peeled in center | All 8 wells were completely peeled |

From Table 5, it was confirmed that, even for easily peelable cells other than nerve cells, the culture method using a cell culture container as in Example 8 in which a first layer including gelatin was layered on the culture surface, a second layer including a polycationic material was layered on the first layer, and a third layer including a cell adhesion factor was layered on that layer made it possible to suppress the peeling of cell aggregates in comparison with the culture method using the cell culture containers of Comparative Examples 17 and 18. In addition, it was also confirmed that it was possible to produce the cell culture container of Example 8 by an inexpensive and simple method.

### [Example 9]

### (Preparation of Cell Culture Container of Example 9)

### «Cell Culture Container, 70% by Mass Ethanol Aqueous Solution, Aqueous Solution Including Gelatin, Aqueous Solution Including Polycationic Material, and Aqueous Solution Including Cell Adhesion Factor»

For the cell culture container, 70% by mass ethanol aqueous solution, aqueous solution including gelatin, aqueous solution including a polycationic material, and aqueous solution including a cell adhesion factor, the same examples as in Example 8 were used.

### <<Preparation of Cell Culture Container of Example 9»

A cell culture container of Example 9 having a modified culture surface was obtained in the same manner as in Comparative Example 8 except that the order of the step of placing an aqueous solution including gelatin in a cell culture container, allowing the container to stand at 37°C for one hour, removing the aqueous solution including gelatin, washing once with ultrapure water, and drying in a safety cabinet and the step of placing an aqueous solution including a polycationic material in a cell culture container, allowing the container to stand at 37°C for one hour, removing the aqueous solution including a polycationic material, washing twice with DPBS and once with ultrapure water, and drying in a safety cabinet, was reversed.

### [Comparative Example 19]

### (Preparation of Cell Culture Container of Comparative Example 19)

### «Cell Culture Container, Aqueous Solution Including Polycationic Material, and Aqueous Solution Including Cell Adhesion Factor»

For the cell culture container, aqueous solution including a polycationic material, and aqueous solution including a cell adhesion factor, the same examples as in Example 9 were used.

### <<Preparation of Cell Culture Container of Comparative Example 19»

A cell culture container of Comparative Example 19 having a modified culture surface was obtained in the same manner as in Example 9, except that the step of bringing an aqueous solution including gelatin into contact with the culture surface of the cell culture container was not performed.

### [Comparative Example 20]

### (Preparation of Cell Culture Container of Comparative Example 20)

### «Cell Culture Container, Aqueous Solution Including Gelatin, and Aqueous Solution Including Cell Adhesion Factor»

For the cell culture container, aqueous solution including gelatin, and aqueous solution including a cell adhesion factor, the same examples as in Example 9 were used.

### <<Preparation of Cell Culture Container of Comparative Example 20»

A cell culture container of Comparative Example 20 having a modified culture surface was prepared in the same manner as in Example 9, except that the step of bringing an aqueous solution including a polycationic material into contact with the culture surface of the cell culture container was not performed.

### [Experiment Example 9]

### (Verification of Peeling of Cell Aggregates other than Nerve Cells)

HEK293 cells were seeded into three wells of each of the cell culture containers of Example 9 and Comparative Examples 19 and 20.

After culturing the HEK293 cells for 3 days, pipetting was performed 4 times, the culture medium was replaced with a culture medium to which a Hoechst staining solution (Hoechst 33342, Thermo Fisher Scientific, Inc.) was added, and each well was observed under a microscope. Furthermore, the average cell coverage area ratio of 3 wells was calculated from images of all wells using imageJ.

The results are shown in Table 6 below.

**Table 6**

| | Example 9 (including gelatin) | Comparative Example 19 (not including gelatin) | Comparative Example 20 (not including polycations) |
|---|---|---|---|
| Cell coverage area ratio after pipetting | 85.2% | 81.1% | 70.3% |

From Table 6, it was confirmed that, even for easily peelable cells other than nerve cells, the culture method using a cell culture container as in Example 9 in which a first layer including a polycationic material was layered on the culture surface, a second layer including gelatin was layered on the first layer, and a third layer including a cell adhesion factor was layered on that layer made it possible to suppress the peeling of cell aggregates in comparison with the culture method using the cell culture containers of Comparative Examples 19 and 20. In addition, it was also confirmed that it was possible to produce the cell culture container of Example 9 by an inexpensive and simple method.

### [Example 10]

### (Preparation of Cell Culture Container of Example 10)

### «Cell Culture Container, Aqueous Solution Including Gelatin, and Aqueous Solution Including Polycationic Material»

For the cell culture container, aqueous solution including gelatin, and aqueous solution including a polycationic material, the same examples as in Example 6 were used.

### <<Preparation of Cell Culture Container of Example 10»

The cell culture container of Example 10 having a modified culture surface was prepared in the same manner as in Example 6, except that the step of bringing an aqueous solution including a cell adhesion factor into contact with the culture surface of the cell culture container was not performed.

### [Comparative Example 21]

### (Preparation of Cell Culture Container of Comparative Example 21)

A cell culture container of Comparative Example 21 having a modified culture surface was obtained using exactly the same method as in Example 10.

### [Experiment Example 10]

### (Verification of Suppression of Agglomeration of Cell Aggregates in 384-Well Plate)

Nerve cells were seeded into four wells of each of the cell culture containers of Example 10 and Comparative Example 21. The same nerve cells as in Experiment Example 7 were used. The cell suspension of Example 10 was mixed with laminin and then seeded therein and the cell suspension of Comparative Example 21 was seeded therein without being mixed with laminin.

After culturing nerve cells for 3 days, each well was observed under a microscope. FIG. 13 shows representative images of the wells in each cell culture container.

From FIG. 13, significant cell agglomeration was observed in the culture method of Comparative Example 21 after 3 days of culturing. On the other hand, in the culture method of Example 10, no significant cell agglomeration was observed. From FIG. 13, it was confirmed that the culture method, as in Example 10, having a first layer including gelatin layered on the culture surface, a second layer including a polycationic material layered on the first layer, and a liquid (culture medium) including a cell adhesion factor layered on that layer made it possible to suppress the agglomeration of cell aggregates in comparison with the culture method not having a liquid (culture medium) including a cell adhesion factor on the layer as in Comparative Example 21. In addition, it was also confirmed that it was possible to produce the cell culture container of Example 10 by an inexpensive and simple method.

From the above results, the present invention provides a cell culture method, a cell culture container, and a method for producing the cell culture container, with which it is possible to suppress the peeling of cell aggregates from a culture surface and obtain stable functional evaluation results.

The present invention includes the following aspects.
[1] A cell culture method including a step of performing adherent culture of cells on a coat layer layered on a surface of a cell culture container by placing a culture medium and the cells in the cell culture container, in which the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with the cells, or the coat layer includes the layer (i), the layer (i) is disposed at a position at which the layer (i) comes into contact with the cells, and the culture medium includes the cell adhesion factor.
[2] The cell culture method according to [1], in which the cell adhesion factor is an extracellular matrix.
[3] The cell culture method according to [1] or [2], in which the gelatin is gelatin derived from fish.
[4] The cell culture method according to any of [1] to [3], in which the polycationic material is polyethyleneimine, polylysine, or polyornithine.
[5] The cell culture method according to any of [1] to [4], in which the cells are easily peelable cells.
[6] A cell culture container, in which a coat layer is layered on a surface, the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with cells during cell culture.
[7] The cell culture container according to [6], in which the cell adhesion factor is an extracellular matrix.
[8] The cell culture container according to [6] or [7], in which the gelatin is gelatin derived from fish.
[9] The cell culture container according to any of [6] to [8], in which the polycationic material is polyethyleneimine, polylysine, or polyornithine.
[10] The cell culture container according to any of [6] to [9], in which the cell culture container is for culturing easily peelable cells.
[11] A method for producing a cell culture container having a modified culture surface, the method including a step 1 of bringing a solution including gelatin or casein into contact with a surface of the cell culture container, a step 2 of removing the solution including the gelatin or the casein, a step 3 of bringing a solution including a polycationic material into contact with the surface of the cell culture container, a step 4 of removing the solution including the polycationic material, a step 5 of bringing a solution including a cell adhesion factor into contact with the surface of the cell culture container, and a step 6 of removing the solution including the cell adhesion factor, in which the production method is performed in the above order, or in an order of the step 3, the step 4, the step 1, the step 2, the step 5, and the step 6.
[12] The production method according to [11], the method further including a step of carrying out a hydrophilic treatment on the surface of the cell culture container before the step 1 when the steps are included in the order of the step 1, the step 2, the step 3, the step 4, the step 5, and the step 6, and further including a step of carrying out a hydrophilic treatment on the surface of the cell culture container before the step 3 when the steps are included in order of the step 3, the step 4, the step 1, the step 2, the step 5, and the step 6.
[13] The production method according to claim 12, in which the step of carrying out the hydrophilic treatment includes a step (a) of bringing a solution including ethanol into contact with the surface of the cell culture container, and a step (b) of removing the solution including the ethanol.
[14] The production method according to any one of [11] to [13], in which the cell adhesion factor is an extracellular matrix.
[15] The production method according to any one of [11] to [14], in which the gelatin is gelatin derived from fish.
[16] The production method according to any one of [11] to [15], in which the polycationic material is polyethyleneimine, polylysine, or polyomithine.
[17] A cell-containing structure including a cell culture container, cells, and a culture medium, in which the culture medium and the cells are placed in the cell culture container, the cell culture container has a coat layer layered on a surface and the cells are adhered on the coat layer, the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with the cells, or the coat layer includes the layer (i), the layer (i) is disposed at a position at which the layer (i) comes into contact with the cells, and the culture medium includes the cell adhesion factor.

The cell culture method according to any one of [1] to [5] above, the cell culture container having a modified culture surface according to any one of [6] to [10] above, the method for producing a cell culture container according to any one of [11] to [16], and the cell-containing structure according to [17] are able to solve the problems in the prior art and achieve the object of the present invention.

It is possible to say that the present invention includes the following aspects.
[P1] A cell culture container having a modified culture surface, the cell culture container including a first layer including gelatin or casein layered on a culture surface of a cell culture container, and a second layer including a polycationic material layered on the first layer.
[P2] The cell culture container according to [P1], further including a third layer layered on the second layer and including a cell adhesion factor.
[P3] The cell culture container according to [P2], in which the cell adhesion factor is an extracellular matrix.
[P4] The cell culture container according to any one of [P1] to [P3], in which the gelatin is gelatin derived from fish.
[P5] The cell culture container according to any one of [P1] to [P4], in which the polycationic material is polyethyleneimine, polylysine, or polyornithine.
[P6] The cell culture container according to any one of [P1] to [P5], in which the cell culture container is for culturing easily peelable cells.
[P7] A method for producing a cell culture container having a modified culture surface, the method including a step of bringing a solution including ethanol into contact with the culture surface of the cell culture container, a step of removing the solution including the ethanol, a step of bringing a solution including gelatin or casein into contact with the surface of the cell culture container, a step of removing the solution including the gelatin or the casein, a step of bringing a solution including a polycationic material into contact with the surface of the cell culture container, and a step of removing the solution including the polycationic material, in this order.
[P8] The production method according to [P7], further including a step of bringing a solution including a cell adhesion factor into contact with the surface of the cell culture container after the step of removing the solution including the polycationic material, and a step of removing the solution including a cell adhesion factor, in this order.
[P9] The production method according to [P8], in which the cell adhesion factor is an extracellular matrix.
[P10] The production method according to any one of [P7] to [P9], in which the gelatin is gelatin derived from fish.
[P11] The production method according to any one of [P7] to [P10], in which the polycationic material is polyethyleneimine, polylysine, or polyornithine.

### [Reference Signs List]

11, 21: Base material
12, 22: Layer including gelatin or casein
13, 23: Layer including polycationic material
14, 24: Layer including cell adhesion factor
15: Easily peelable cells
16: Liquid including easily peelable cells and cell adhesion factor

### [Citation List]

### [Patent Document]

[Patent Document 1]
Japanese Unexamined Patent Application, First Publication No. 2018-166477

### [Non Patent Documents]

[Non Patent Document 1]
   "Singlewell-M64-GLx_datasheet", Internet <URL: http://www.wpi-europe.com/downloads/content/Singlewell-M64-GLx_datasheet.pdf> [Retrieved on 2022/01/14]
[Non Patent Document 2]
   "Microelectrode Array (MEA) Manual", Internet <URL: https://www.multichannelsystems.com/sites/multichannelsystems.com/files/documents/m anuals/MCS_MEA_Manual.pdf> [Retrieved on 2022/01/ 14]
[Non Patent Document 3]
   "Primary culture of hippocampus-derived nerve cells", Internet <URL: https://alphamedsci.com/common/pdf/dissociatedl_190621%20(J).pdf> [Retrieved on 2021/01/14]

## Claims

1. A cell culture method comprising:
a step of performing adherent culture of cells on a coat layer layered on a surface of a cell culture container by placing a culture medium and the cells in the cell culture container,
wherein the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with the cells, or
the coat layer includes the layer (i), the layer (i) is disposed at a position at which the layer (i) comes into contact with the cells, and the culture medium includes the cell adhesion factor.

2. The cell culture method according to claim 1,
wherein the cell adhesion factor is an extracellular matrix.

3. The cell culture method according to claim 1 or 2,
wherein the gelatin is gelatin derived from fish.

4. The cell culture method according to any one of claims 1 to 3,
wherein the polycationic material is polyethyleneimine, polylysine, or polyornithine.

5. The cell culture method according to any one of claims 1 to 4,
wherein the cells are easily peelable cells.

6. A cell culture container,
wherein a coat layer is layered on a surface, the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with cells during cell culture.

7. The cell culture container according to claim 6,
wherein the cell adhesion factor is an extracellular matrix.

8. The cell culture container according to claim 6 or 7,
wherein the gelatin is gelatin derived from fish.

9. The cell culture container according to any one of claims 6 to 8,
wherein the polycationic material is polyethyleneimine, polylysine, or polyornithine.

10. The cell culture container according to any one of claims 6 to 9, wherein the cell culture container is for culturing easily peelable cells.

11. A method for producing a cell culture container having a modified culture surface, the method comprising:
a step 1 of bringing a solution including gelatin or casein into contact with a surface of the cell culture container;
a step 2 of removing the solution including the gelatin or the casein;
a step 3 of bringing a solution including a polycationic material into contact with the surface of the cell culture container;
a step 4 of removing the solution including the polycationic material;
a step 5 of bringing a solution including a cell adhesion factor into contact with the surface of the cell culture container; and
a step 6 of removing the solution including the cell adhesion factor,
wherein the production method is performed in the above order, or in an order of the step 3, the step 4, the step 1, the step 2, the step 5, and the step 6.

12. The production method according to claim 11, the method further comprising:
a step of carrying out a hydrophilic treatment on the surface of the cell culture container before the step 1 when the steps are included in the order of the step 1, the step 2, the step 3, the step 4, the step 5, and the step 6; and
a step of carrying out a hydrophilic treatment on the surface of the cell culture container before the step 3 when the steps are included in order of the step 3, the step 4, the step 1, the step 2, the step 5, and the step 6.

13. The production method according to claim 12,
wherein the step of carrying out the hydrophilic treatment includes a step (a) of bringing a solution including ethanol into contact with the surface of the cell culture container, and a step (b) of removing the solution including the ethanol.

14. The production method according to any one of claims 11 to 13,
wherein the cell adhesion factor is an extracellular matrix.

15. The production method according to any one of claims 11 to 14,
wherein the gelatin is gelatin derived from fish.

16. The production method according to any one of claims 11 to 15,
wherein the polycationic material is polyethyleneimine, polylysine, or polyornithine.

17. A cell-containing structure comprising:
a cell culture container;
cells; and
a culture medium,
wherein the culture medium and the cells are placed in the cell culture container,
the cell culture container has a coat layer layered on a surface and the cells are adhered on the coat layer,
the coat layer includes a layer (i) that includes a layer (i-1) including gelatin or casein and a layer (i-2) that includes a polycationic material and a layer (ii) that is layered on the layer (i) and includes a cell adhesion factor, and the layer (ii) is disposed at a position at which the layer (ii) comes into contact with the cells, or
the coat layer includes the layer (i), the layer (i) is disposed at a position at which the layer (i) comes into contact with the cells, and the culture medium includes the cell adhesion factor.
